(19) Europäisches Patentamt
European Patent Office
Office européen des brevets

(11) Numéro de publication: **0 313 458 B1**

(12) # FASCICULE DE BREVET EUROPEEN

(45) Date de publication de fascicule du brevet: **11.08.93**

(51) Int. Cl.[5]: **C07D 513/04**, A61K 31/425, //(C07D513/04,277:00,235:00)

(21) Numéro de dépôt: **88402634.5**

(22) Date de dépôt: **19.10.88**

(54) **Nouveaux imidazo (2,1-b) benzothiazoles et leurs sels d'addition avec les acides, leur procédé et les intermédiaires de préparation, leur application comme médicaments et les compositions pharmaceutiques les renfermant.**

(30) Priorité: **20.10.87 GB 8724566**

(43) Date de publication de la demande: **26.04.89 Bulletin 89/17**

(45) Mention de la délivrance du brevet: **11.08.93 Bulletin 93/32**

(84) Etats contractants désignés: **AT BE CH DE ES FR GR IT LI LU NL SE**

(56) Documents cités:
**FR-A- 2 260 344**

**JOURNAL OF MEDICINAL CHEMISTRY, vol. 31, no. 6, juin 1988, pages 1220-1226, American Chemical Society, Washington, DC, US; S. CLEMENTS-JEWERY et al.: "(Imidazo[1,2-a]pyrimidin-2-yl)phenylmethanones and related compounds as potential nonsedative anxiolytics"**

(73) Titulaire: **ROUSSEL-UCLAF**
**35, Boulevard des Invalides**
**F-75007 Paris(FR)**

(72) Inventeur: **Hedgecock, Charles John Robert**
**186 High Street**
**Wootton Bassett Wiltshire SN4 7BZ(GB)**
Inventeur: **Kay, David Paul**
**4, Church Path**
**Purton-Swindon Wiltshire(GB)**

(74) Mandataire: **Vieillefosse, Jean-Claude et al**
**Roussel-Uclaf 111, route de Noisy B.P. 9**
**F-93230 Romainville (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.6/3.3.1)

## Description

La présente invention concerne de nouveaux imidazo [2,1-b] benzothiazoles et leurs sels d'addition avec les acides, ainsi que le procédé et les intermédiaires de préparation, l'application à titre de médicaments de ces nouveaux produits et les compositions les renfermant.

L'invention a pour objet de nouveaux imidazo [2,1-b] benzothiazoles répondant a la formule générale (I) :

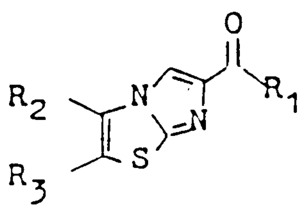

(I)

dans laquelle $R_1$ représente un groupement de formule (A) :

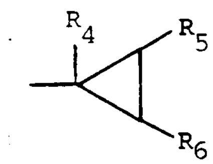

(A)

dans laquelle $R_4$ représente un atome d'hydrogène ou un radical alcoyle renfermant de 1 à 5 atomes de carbone, $R_5$ représente un atome d'hydrogène ou un radical alcoyle renfermant de 1 à 5 atomes de carbone, $R_6$ représente un atome d'hydrogène, un radical alcoyle renfermant de 1 à 5 atomes de carbone, un radical phényle, un atome d'halogène, un radical alkoxycarbonyle renfermant de 2 à 5 atomes de carbone, un radical cyano, un radical amido, un radical mono ou dialcoylamido dont les groupements alcoyles renferment de 1 à 5 atomes de carbone ou $R_1$ représente un groupement de formule (B) :

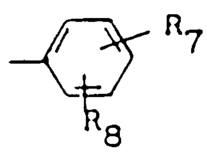

(B)

dans laquelle $R_7$ et $R_8$, identiques ou différents, représentent un atome d'hydrogène,un radical alcoyle, linéaire ou ramifié, renfermant de 1 à 5 atomes de carbone, $R_2$ et $R_3$ représentent un groupement :

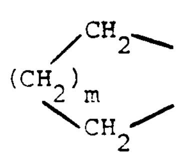

ou

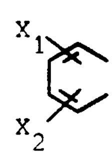

dans lequel m représente le nombre 1, 2 ou 3, $X_1$ et $X_2$, identiques ou différents, représentent un atome d'hydrogène, un radical alcoyle linéaire ou ramifié, renfermant de 2 à 5 atomes de carbone, un radical alkoxy, linéaire ou ramifié, renfermant de 1 à 5 atomes de carbone, un radical aralkoxy, un radical aryloxy, un atome d'halogène, un groupement nitrile ou azido, ou $X_1$ et $X_2$ forment ensemble un radical méthylènedioxy, ainsi que leurs sels d'addition avec les acides minéraux ou organiques.

Dans la formule générale (I) et dans ce qui suit

- par un radical alcoyle renfermant de 1 à 5 atomes de carbone, on entend, de préférence un radical méthyle, éthyle, propyle, butyle, pentyle, isopropyle, isobutyle ou terbutyle ;
- par atome d'halogène, on entend de préférence un atome de fluor, de chlore ou de brome ;
- par radical alcoxycarbonyle renfermant de 2 à 5 atomes de carbone, on entend de préférence un radical méthoxycarbonyle, éthoxycarbonyle ou propoxy carbonyle ;

- par radical mono ou dialcoylamido dont les radicaux alcoyles renfermant de 1 à 5 atomes de carbone, on entend de préférence un radical monométhylamido, diméthylamido, monoéthylamido, diéthylamido, monopropylamido ou dipropylamido ;
- par radical alkényle renfermant de 2 à 5 atomes de carbone, on entend de préférence un radical vinyle, allyle, 3-butétnyle ou isopropényle ;
- par radical alkoxy renfermant de 1 à 5 atomes de carbone, on entend de préférence un radical méthoxy, éthoxy, propoxy ou isopropoxy ;
- par radicaux aralkoxy, on entend de préférence un radical benzyloxy, phényléthyloxy, thiénylméthyloxy ;
- par radicaux aryloxy, on entend de préférence un radical phényloxy ou naphtyloxy.

Les sels d'addition avec les acides minéraux ou organiques peuvent être, par exemple, les sels formés avec les acides chlorhydrique, bromhydrique, iodhydrique, nitrique, sulfurique, phosphorique, propionique, formique, benzoïque, maléïque, fumarique, succinique, tartrique, citrique, oxalique, glyoxylique, aspartique, alcanesulfoniques tels que les acides méthanesulfoniques et arylsulfonique, tel que l'acide benzènesulfonique.

Parmi les produits, objet de l'invention, on peut citer notamment les dérivés répondant à la formule (I) ci-dessus, ainsi que leurs sels, caractérisé en ce que dans ladite formule (I), $R_1$ représente soit un groupement de formule (A) dans laquelle $R_4$, $R_5$ et $R_6$ représentent un atome d'hydrogène, soit un groupement de formule (B) dans laquelle $R_7$ et $R_8$ représentent un atome d'hydrogène et $R_2$ et $R_3$ ensemble représentent un groupement

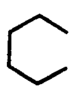

ou

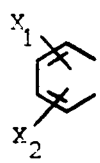

dans lequel $X_1$ et $X_2$, identiques ou différents, représentent un atome d'hydrogène, un radical méthyle, un radical méthoxy, un radical isopropoxy ou un radical benzyloxy.

Parmi ces derniers, on peut citer les dérivés répondant à la formule (I) ci-dessus ainsi que leurs sels, caractérisés en ce que dans ladite formule (I), $R_1$ représente un groupement de formule (A) dans laquelle $R_4$, $R_5$ et $R_6$ représentent un atome d'hydrogène et $R_2$ et $R_3$ ensemble représentent un groupement

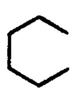

ou

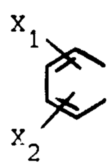

dans lequel $X_1$ représente un atome d'hydrogène et $X_2$ représente un radical méthoxy, un radical isopropoxy ou un radical benzyloxy.

Parmi les produits, objet de l'invention, on retient tout particulièrement ceux dont les noms suivent :

- la (7-isopropoxyimidazo [2,1-b] benzothiazol-2-yl) cyclopropylméthanone et ses sels,
- la (7-méthoxyimidazo [2,1-b] benzothiazol-2-yl) cyclopropylméthanone et ses sels,
- la (7-benzyloxyimidazo [2,1-b] benzothiazol-2-yl) cyclopropylméthanone,
- la (5,6,7,8-tétrahydroimidazo [2,1-b] benzothiazol-2-yl) cyclopropyl méthanone et leurs sels d'addition avec les acides.

L'invention a également pour objet un procédé de préparation des nouveaux imidazo [2,1-b] benzothiazoles répondant à la formule ($I_A$) :

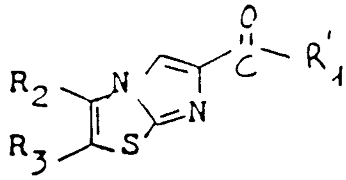

($I_A$)

dans laquelle $R_2$ et $R_3$ ont la signification déjà indiquée et $R'_1$ représente un groupement de formule (A) dans lequel $R_4$ représente un atome d'hydrogène et $R_5$ ou $R_6$ représentent un atome d'hydrogène ou un radical alcoyle renfermant de 1 à 5 atomes de carbone ou $R'_1$ représente un groupement de formule (B) dans lequel $R_7$ et $R_8$ ont la signification indiquée, caractérisé en ce que soit l'on fait réagir un produit de formule (VIII) :

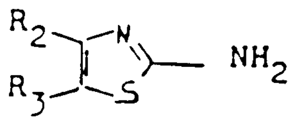

(VIII)

dans laquelle $R_2$ et $R_3$ ont la signification déjà indiquée avec du 3-bromo 1-hydroxypyran-2-one pour obtenir un produit de formule (VII) :

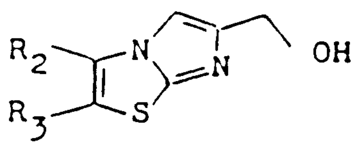

(VII)

dans laquelle $R_2$ et $R_3$ ont la signification déjà indiquée, soit l'on réduit un produit de formule (IX) :

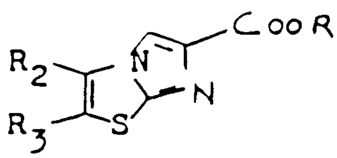

(IX)

dans laquelle $R_2$ et $R_3$ ont la signification déjà indiquée et R représente un radical alcoyle renfermant de 1 à 3 atomes de carbone, pour obtenir un produit de formule (VII) telle que définie ci-dessus, produit de formule (VII) que l'on soumet à un agent d'oxydation pour obtenir un produit de formule (V) :

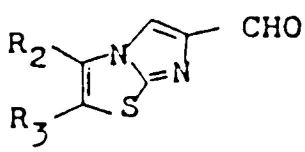

(V)

dans laquelle $R_2$ et $R_3$ ont la signification déjà indiquée que l'on fait réagir avec un produit de formule (VI) :

$M-R'_1$ (VI)

dans laquelle M représente un atome de métal alcalin lithium ou un reste

- Mg Hal dans lequel Hal représente un arome de chlore, de brome ou d'iode et $R_1$ a la signification déjà indiquée pour obtenir un produit de formule (IV) :

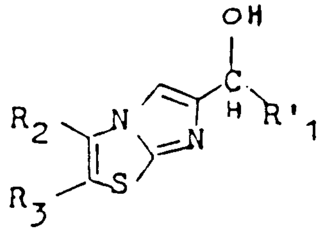

(IV)

dans laquelle $R'_1$, $R_2$ et $R_3$ ont la signification déjà indiquée, puis oxyde le produit de formule (IV) ainsi obtenu, pour obtenir le produit de formule ($I_A$) que l'on peut salifier le cas échéant.

Dans des conditions préférentielles de mise en oeuvre de l'invention, le procédé de préparation ci-dessus décrit est caractérisé en ce que :

- la réaction du produit de formule (V) avec le produit de formule (VI) est effectuée dans des conditions anhydres, dans un solvant organique tel que le tétrahydrofuranne,
- l'oxydation du produit de formule (IV) est effectuée avec le dioxyde de manganèse, l'acide nitrique, le chlorure ferrique ou l'oxyde de chrome en présence de pyridine ou encore par la méthode d'Oppenauer ou enfin par déshydrogénation en prsence d'un catalyseur à base de cuivre,
- l'oxydation du produit de formule (VII) peut avantageusement être effectuée au moyen de dioxyde de manganèse,
- la réduction du produit de formule (IX) peut avantageusement être effectuée au moyen du borohydrure de lithium.

Les 2-aminobenzothiazoles de formule (VIII) peuvent être préparés comme indiqué dans J. Het. Chem. (1980), 17, 1325. Les 2-amino 4,5,6,7-tétrahydrobenzothiazoles de formule (VIII) peuvent être préparés comme indiqué dans J. Gem. Chem. USSR. 16, 1701-5 (1946).

Les produits de formule (IX) peuvent être préparés comme indiqué dans Farmaco. Ed. Sci. (1977), 32, 735.

L'invention a aussi pour objet un procédé de préparation des nouveaux imidazo [2,1-b] benzothiazoles répondant à la formule ($I_B$) :

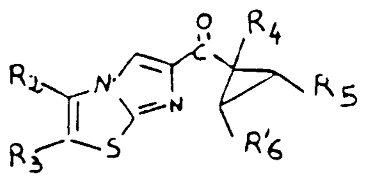

($I_B$)

dans laquelle $R_2$, $R_3$, $R_4$ et $R_5$ ont la signification déjà indiquée et $R'_6$ représente un atome d'hydrogène, un atome d'halogène, un radical alcoyle renfermant de 1 à 5 atomes de carbone, un radical alkoxycarbonyle renfermant de 2 à 5 atomes de carbone, un radical phényle, un radical cyano ou un groupement

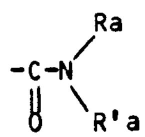

dans lequel Ra et R'a, identiques ou différents, représentent un atome d'hydrogène ou un radical alcoyle renfermant de 1 à 5 atomes de carbone, caractérisé en ce que l'on fait réagir un produit (V) telle que défini ci-dessus avec un produit de formule (XII) :

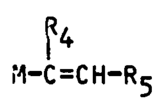

(XII)

dans laquelle M, $R_4$ et $R_5$ ont la signification déjà indiquée pour obtenir un produit de formule (XI) :

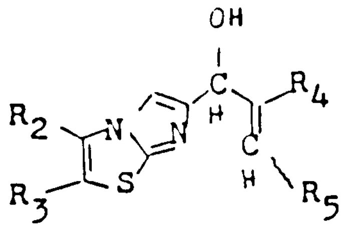

(XI)

dans laquelle $R_2$, $R_3$, $R_4$ et $R_5$ ont la signification déjà indiquée, pour oxyder le produit de formule (XI), pour obtenir un produit de formule (X) :

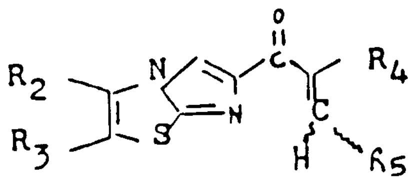

(X)

dans laquelle $R_2$, $R_3$, $R_4$ et $R_5$ ont la signification déjà indiquée, puis fait réagir ce dernier avec un agent de cyclisation approprié, pour obtenir un produit de formule ($I_B$) dans laquelle $R_2$, $R_3$, $R_4$ et $R_5$ ont la signification déjà indiquée et $R'_6$ représente un atome d'hydrogène, un atome d'halogène, un radical alcoyle renfermant de 1 à 5 atomes de carbone, un radical alcoxycarbonyle contenant de 2 à 5 atomes de carbone ou un radical phényle, puis saponifie le cas échéant le produit obtenu dans lequel $R'_6$ représente un radical alcoxycarbonyle pour obtenir un produit de formule (XIV) :

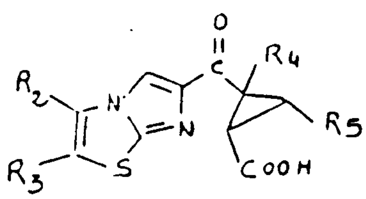

(XIV)

dans laquelle $R_2$, $R_3$, $R_4$ et $R_5$ ont la signification déjà indiquée, puis fait réagir ledit produit de formule (XIV) avec une amine de formule (XIII) :

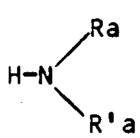

(XIII)

dans laquelle Ra et R'a ont la signification déjà indiquée pour obtenir le produit correspondant de formule ($I_B$) dans laquelle $R'_6$ représente un groupement

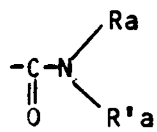

puis le cas échéant, déshydrate ce dernier dans lequel Ra et R'a représentent un atome d'hydrogène pour obtenir le produit correspondant de formule ($I_B$) dans laquelle $R'_6$ représente un radical cyano, et le cas échéant salifie les produits de formule ($I_B$) ainsi obtenus.

Dans des conditions préférentielles de mise en oeuvre de l'invention, le procédé de préparation ci-dessus est caractérisé en ce que :

- la réaction du produit de formule (V) avec le produit de formule (XII) est effectuée dans des conditions anhydres, dans un solvant organique tel que le tétrahydrofuranne,
- l'oxydation du produit de formule (XI) est effectuée de préférence avec le dioxyde de manganèse, l'acide nitrique, le chlorure ferrique ou l'oxyde de chrome, en présence de pyridine, ou encore par la méthode d'Oppenauer ou enfin par déshydrogénation en présence d'un catalyseur à base de cuivre,
- l'agent de cyclisation approprié est un réactif capable d'introduire le groupement CH-$R'_6$ au niveau de la double liaison,
- lorsque l'on désire obtenir un produit de formule ($I_B$) dans laquelle $R_4$ représente un radical alcoyle renfermant de 1 à 5 atomes de carbone, $R_5$ représente un atome d'hydrogène et $R'_6$ représente un atome d'hydrogène ou un radical alcoyle renfermant de 1 à 5 atomes de carbone, la cyclisation est avantageusement effectuée au moyen d'un iodure de trialkyloxosulphonium en opérant au sein d'un solvant organique tel que le diméthylformamide,
- lorsque l'on désire obtenir un produit de formule ($I_B$) dans laquelle $R_4$ représente un atome d'hydrogène, $R_5$ représente un radical alcoyle renfermant de 1 à 3 atomes de carbone et $R'_6$ représente un atome d'hydrogène cu un radical alcoyle renfermant de 1 à 5 atomes de carbone, la cyclisation est avantageusement effectuée au moyen d'un tétrafluoroborate de diméthylaminoalkylphényloxosulfonium en opérant au sein d'un solvent organique tel que le diméthylformamide,
- lorsque l'on désire obtenir un produit de formule ($I_B$) dans laquelle $R_4$ et $R_5$ représentent un atome d'hydrogène et $R'_6$ représente un groupement alcoxycarbonyle, le cyclisation est avantageusement effectuée au moyen d'un acétate d'alcoyle de diméthylsulfuranylidène ou d'un anion benzyl diméthylsulfonium en opérant au sein d'un solvant organique tel que le chloroforme,
- lorsque l'on désire obtenir un produit de formule ($I_B$) dans laquelle $R_4$ et $R_5$ représentent un atome d'hydrogène et $R'_6$ représente un atome d'halogène, la cyclisation est avantageusement effectuée au moyen d'un halogéno-méthylylidène de diméthylaminophénylsulfonium en opérant au sein d'un solvant organique tel que le diméthylformamide,
- la saponification du produit de formule ($I_B$) dans laquelle $R'_6$ représente un radical alkoxycarbonyle est effectuée de préférence au moyen d'un hydroxyde alcalin tel que l'hydroxyde de sodium,
- la réaction du produit de formule (XIV) avec l'amine de formule (XIII) est effectuée de préférence au sein d'un solvant organique anhydre en présence de carbonyldiimidazole,
- la déshydratation du produit de formule ($I_B$) est effectuée de préférence au moyen d'un anhydride d'acide fort tel que l'anhydride d'acide trifluoroacétique au sein d'un solvant organique tel que le dichlorométhane.

Les produits de formule (I) présentent un caractère basique.

On peut avantageusement préparer les sels d'addition des produits de formule (I) en faisant réagir, en proportions sensiblement stoéchiométriques, un acide minéral ou organique avec lesdits produits de formule (I). Les sels peuvent être préparés sans isoler les bases correspondantes.

Les produits, objet de la présente invention, possèdent de très intéressantes propriétés pharmacologiques ; on note en particulier des propriétés agonistes inverses des benzodiazépines faibles à fortes suivant la substitution.

Certains produits présentent, en outre, des propriétés tranquillisantes.

Ces propriétés sont illustrées plus loin dans la partie expérimentale.

Ces propriétés justifient l'utilisation des nouveaux imidazo [2,1-b] benzothiazoles de formule (I) ainsi que de leurs sels pharmaceutiquement acceptables, à titre de médicaments. La présente invention a ainsi également pour objet l'application à titre de médicaments des nouveaux imidazo [2,1-b] benzothiazoles tels que définis par la formule générale (I), ainsi que de leurs sels d'addition avec les acides pharmaceutiquement acceptables.

Parmi les médicaments, objet de l'invention, on retient notamment les médicaments caractérisés en ce qu'ils sont constitués par les nouveaux imidazo [2,1-b] benzothiazoles répondant à la formule (I) dans laquelle $R_1$ représente un groupement de formule (A) dans laquelle $R_4$, $R_5$ et $R_6$ représentent un atome d'hydrogène et $R_1$ représente un groupement de formule (B) dans laquelle $R_7$ et $R_8$ représentent un atome d'hydrogène et $R_2$ et $R_3$ représentent un groupement

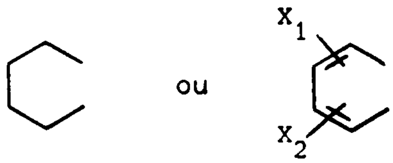

dans lequel $X_1$ et $X_2$, identiques ou différents représentent un atome d'hydrogène, un radical méthyle, un radical méthoxy, un radical isopropoxy ou un radical benzyloxy, ainsi que par leurs sels d'addition avec les acides pharmaceutiquement acceptables.

Parmi les médicaments, objet de l'invention, on retient tout particulièrement ceux répondant à la formule (I), caractérisés en ce que dans ladite formule (I), $R_1$ représente un groupement de formule (A) dans laquelle $R_4$, $R_5$ et $R_6$ représentent un atone d'hydrogène et $R_2$ et $R_3$ représentent un groupement

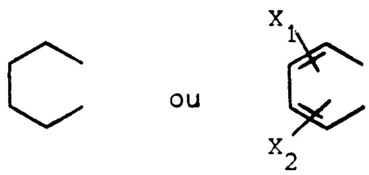

dans lequel $X_1$ représente un atome d'hydrogène et $X_2$ représente un radical méthoxy, un radical isopropyloxy ou un radical benzyloxy ainsi que leurs sels d'addition avec les acides pharmaceutiquement acceptables.

Parmi les médicaments préférés de l'invention, on retient tout particulièrement :

- la (7-isopropoxyimidazo [2,1-b] benzothiazol-2-yl) cyclopropylméthanone,
- la (7-méthoxyimidazo [2,1-b] benzothiazol-2-yl) cyclopropylméthanone,
- la (7-benzyloxyimidazo [2,1-b] benzothiazol-2-yl) cyclopropylméthanone,
- la 5,6,7,8-tétrahydroimidazo [2,1-b] benzothiazol-2-yl) cyclopropylméthanone,

ainsi que leurs sels pharmaceutiquement acceptables.

Ces médicaments trouvent, par exemple, leur emploi dans le traitement des troubles de la mémoire, notamment en gériatrie, des troubles de la sénescence cérébrale. Certains produits peuvent également être utilisés dans le traitement de l'obésité ainsi que comme tranquillisants mineurs dans le traitement de certaines agitations ou irratabilité.

La dose usuelle, variable selon le produit utilisé, le sujet traité et l'affection en cause peut être par exemple, de 0,1 mg à 200 mg par jour, par voie orale.

L'invention a également pour objet les compositions pharmaceutiques qui renferment au moins un dérivé précité ou l'un de ses sels pharmaceutiquement acceptables à titre de principe actif.

A titre de médicaments, les dérivés répondant à la formule (I) et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés, simples ou dragéifiés, les gélules, les capsules, les granulés, les suppositoires, les préparations injectables ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les divers agents mouillants, dispersants ou émulsifiants, les conservateurs.

L'invention a enfin pour objet, à titre de produits nouveaux et notamment d'intermédiaires nécessaires à la préparation des produits de formule (I) :

- les produits de formule (IV) :

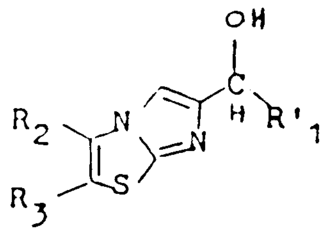

(IV)

dans laquelle R'$_1$, $R_2$ et $R_3$ ont la signification déjà indiquée ;

- les produits de formule (XI) :

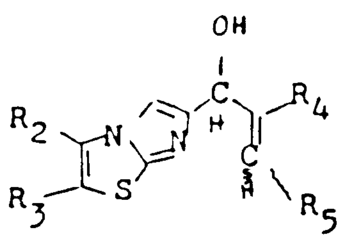

(XI)

dans laquelle $R_1$, $R_2$, $R_3$, $R_4$ et $R_5$ ont la signification déjà indiquée ;

- les produits de formule (X) :

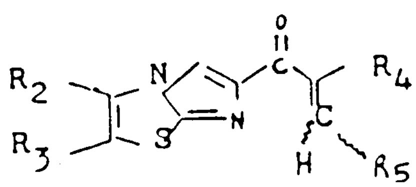

(X)

dans laquelle $R_2$, $R_3$, $R_4$ et $R_5$ on la signification déjà indiquée ;

- les produits de formule (XIV) :

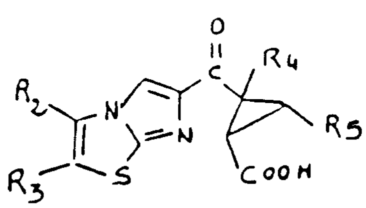

(XIV)

dans laquelle $R_2$, $R_3$, $R_4$ et $R_5$ ont la signification déjà indiquée.

Il va être donné maintenant des exemples de mise en oeuvre de l'invention.

**Exemple 1 : (7-méthoxyimidazo [2,1-b] benzothiazol-2-yl) cyclopropylméthanone.**

**Stade A :** (7-méthoxyimidazo [2,1-b] benzothiazol-2-yl) cyclopropyl méthanol.

On met en suspension 3,9 g de 7-méthoxyimidazo [2,1-b] benzothiazol-2-carboxaldéhyde dans 80 cm3 de tétrahydrofuranne puis ajoute une solution de bromure de cyclopropyl magnésium (préparé à partir de 3,6 g de bromure de cyclopropyle et 0,8 mg de magnésium) dans 50 cm3 de tétrahydrofuranne. On agite le mélange à température ambiante pendant 3 heures, verse dans une solution aqueuse saturée en chlorure d'ammonium. On extrait à l'acétate d'éthyle, sèche sur sulfate de mégnésien et évapore sous pression

réduite. On chromatographie le résidu sur silice (éluant : chlorure de méthylène à 2% de méthanol) et obtient 2,75 g de 7-méthoxyimidazo [2,1-b] benzothiazol-2-yl cyclopropyl méthanol. F = 151-153¤C (Ethanol).

| Analyse : $C_{14}H_{14}N_2O_2S$ | | | | | | | | |
|---|---|---|---|---|---|---|---|---|
| Calculé :<br>Trouvé : | C% | 61,30<br>61,17 | H% | 5,14<br>5,23 | N% | 10,21<br>10,24 | S% | 11,69<br>11,48 |

**Stade B :** (7-méthoxyimidazo [2,1-b] benzothiazol-2-yl) cyclopropylméthanone.

On chauffe 1 heure au reflux 1,63 g de produit obtenu au stade A et 4,8 g de dioxyde de manganèse dans 300 cm3 de chloroforme. On filtre à chaud et concentre à sec. On triture le résidu dans l'éther et obtient 1,45 g de produit attendu. F = 213-214¤C (Ethanol).

| Analyse : $C_{14}H_{12}N_2O_2S$ | | | | | | | | |
|---|---|---|---|---|---|---|---|---|
| Calculé :<br>Trouvé : | C% | 61,75<br>62,07 | H% | 4,44<br>4,49 | N% | 10,29<br>10,32 | S% | 11,77<br>11,76 |

**Exemples 2 à 8 :**

En utilisant une méthode analogue à celle de l'exemple mais en utilisant au départ les composés correspondants de formule (V) dans laquelle $R_1$ et $R_2$ ont les significations indiquées dans le tableau I ci-après, on a préparé les composés des exemples 2 à 8.

Les analyses spectrométriques, les rendements, les points de fusion et les résultats de la microanalyse de ces composés sont donnés dans le tableau I.

**Exemple 2 : imidazo [2,1-b] benzothiazol-2-yl cyclopropyl méthanone.**

**Exemple 3 : 6-méthoxyimidazo [2,1-b] benzothiazol-2-yl cyclopropyl méthanone.**

**Exemple 4 : 7-benzyloxyimidazo [2,1-b] benzothiazol-2-yl cyclopropyl méthanone.**

**Exemple 5 : 6,7-méthylènedioxyimidiazo [2,1-b] benzothiazol-2-yl cyclo propylméthanone.**

**Exemple 6 : 5,6,7,8-tétrahydroimidazo [2,1-b] benzothiazol-2-yl cyclopropyl méthanone.**

**Exemple 7 : 7-isopropoxyimidazo [2,1-b] benzothiazol-2-yl cyclopropyl méthanone.**

**Exemple 8 : 6,7-diméthylimidazo [2,1-b] benzothiazol-2-yl cyclopropyl méthanone.**

TABLEAU I

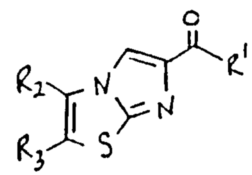

| Ex. | Rendt. % | $R^1$ | $R^2$ $R^3$ | F°C | Spectre IR $cm^{-1}$ | Calculé Trouvé | Analyse C% | H% | N% | S% |
|---|---|---|---|---|---|---|---|---|---|---|
| 1 | 89 | 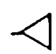 | 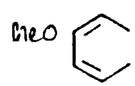 | 213-4 | 3150 ($C_1H$), 1649 (C=O), 1618, 1518, 1507, 1488, 1377 | $C_{14}H_{12}N_2O_2S$ | 62.01<br>61.75 | 4.49<br>4.44 | 10.32<br>10.29 | 11.76<br>11.77 |
| 2 | 92 | 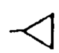 | 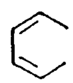 | 204-5 | 3160 ($C_1H$), 1650 (C=O), 1518 1502, 1374, 1209, 1050 | $C_{13}H_{10}N_2OS$ | 64.18<br>64.44 | 4.22<br>4.16 | 11.51<br>11.56 | 13.25<br>13.23 |
| 3 | 85 | 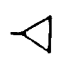 | 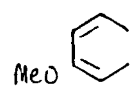 | 235-6 | 3140 ($C_1H$), 1651 (C=O), 1587, 1524br, 1423, 1374, 1286 | $C_{14}H_{12}N_2O_2S$ | 61.48<br>61.76 | 4.51<br>4.44 | 10.22<br>10.29 | 11.67<br>11.77 |
| 4 | 91 | 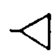 | 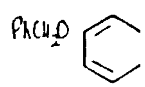 | 195-7 | 3160 ($C_1H$), 1651 (C=O), 1519, 1501, 1481, 1280, 1197 | $C_{20}H_{16}N_2O_2S$ | 68.70<br>68.95 | 4.70<br>4.63 | 8.<br>8.04 | 9.15<br>9.20 |

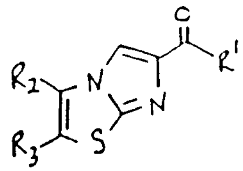

TABLEAU I (Suite)

| Ex. | Rendt. % | $R^1$ | $R^2$ $R^3$ | F°C | Spectre IR $cm^{-1}$ | Analyse Calculé Trouvé | C% | H% | N% | S% |
|---|---|---|---|---|---|---|---|---|---|---|
| 5 | 75 | 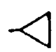 | 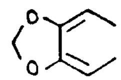 | 304-7 | 3140 ($C_1H$), 1655 (C=O), 1520, 1500, 1466, 1381, 1254 | $C_{14}H_{10}N_2O_3S$ | 58.73 | 3.52 | 9.78 | 11.2C |
| 6 | 85 | 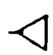 | 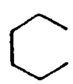 | 182-4 | 3100 ($C_1H$), 1650 (C=O), 1512, 1443, 1378, 1311, 1234 | $C_{13}H_{14}N_2OS$ | 63.52<br>63.39 | 5.78<br>5.73 | 11.59<br>11.73 | 12.95<br>13.02 |
| 7 | 85 | 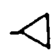 | 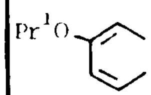 | 148-50 | 3120 ($C_1H$), 1650 (C=O), 1610, 1516, 1497, 1475, 1370, 1290 | $C_{16}H_{16}N_2O_2S$ | 63.82<br>63.98 | 5.45<br>5.37 | 9.24<br>9.33 | 10.71<br>10.67 |
| 8 | 81 | 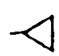 | 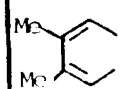 | 286-8 | 3120 ($C_1H$), 1639(C=O), 1500, 1362, 1295, 1187, 1160 | $C_{15}H_{14}N_2OS$ | 66.47<br>66.64 | 5.29<br>5.22 | 10.30<br>10.36 | 11.89<br>11.86 |

**Préparation du 7-méthoxyimidazo [2,1-b] benzothiazol-2-carboxaldéhyde utilisé au départ de l'exemple 1.**

a) 7-méthoxyimidazo [2,1-b] benzothiazol-2-méthanol.

On agite pendant 3 heures à 80¤C et sous atmosphère d'azote 11,6 g de 2-amino 5-méthoxybenzothiazole et 11,6 g de 3-bromo 1-hydroxypropan-2-one dans 120 cm3 de tétrahydrofuranne. On refroidit et sépare par décantation le liquide surnageant. On chauffe le résidu dans 60 cm3 d'éthanol pendant 1 heure, refroidit, filtre et obtient l'hydrobromure du 7-méthoxyimidazo [2,1-b] benzothiazol-2-méthanol que l'on dissout dans l'eau, alcalinise avec une solution aqueuse de bicarbonate de sodium, extrait au chloroforme, sèche et concentre sous pression réduite. On reprend le résidu dans l'éther éthylique et obtient 6,3 g de produit attendu. F = 202-205¤C (éthanol).

| Analyse : $C_{11}H_{10}N_2O_2S$ | | | | | | |
|---|---|---|---|---|---|---|
| Calculé :<br>Trouvé : | C% | 56,40<br>56,32 | H% | 4,30<br>4,36 | N% | 11,96<br>11,93 |

b) 7-méthoxyimidazo [2,1-b] benzothiazol)2-carboxaldéhyde.

On chauffe 2 heures au reflux 5 g de produit obtenu au stade a) et 10 g de dioxyde de manganèse dans 530 cm3 de chloroforme. On filtre à chaud sur célité et évapore sous pression réduite. On obtient 3,92 g de produit attendu. F = 214-215¤C (éthanol).

| Analyse : $C_{11}H_8N_2O_2S$ | | | | | | | | |
|---|---|---|---|---|---|---|---|---|
| Calculé :<br>Trouvé : | C% | 56,89<br>56,79 | H% | 3,47<br>3,56 | N% | 12,06<br>12,08 | S% | 13,80<br>13,75 |

**Préparation du 6-méthoxyimidazo [2,1-b] benzothiazol-2-méthanol utilisé au départ de l'exemple 3.**

On agite 48 heures à température ambiante sous atmosphère d'azote, 4 g d'imidazo [2,1-b] benzothiazol-2-carboxylate d'éthyle et 1,5 g de borohydrure de lithium dans le tétrahydrofuranne. On trite le mélange avec de l'acide chlorhydrique (2N) en excès afin de décomposer le complexe. On alcalinise le milieu réactionnel à l'aide d'une solution aqueuse de bicarbonate de sodium, extrait à l'acétate d'éthyle, sèche et concentre à sec sous pression réduite. On obtient 3,27 g de produit attendu. F = 189-190¤C (éthanol).

| Analyse : $C_{11}H_{10}N_2O_2S$ | | | | | | | | |
|---|---|---|---|---|---|---|---|---|
| Calculé :<br>Trouvé : | C% | 56,40<br>56,37 | H% | 4,30<br>4,38 | N% | 11,96<br>11,90 | S% | 13,69<br>13,65 |

**Exemple 9** :

On a préparé des comprimés répondant à la formulation suivante :

| | |
|---|---|
| - Produit de l'exemple 1<br>- Excipient pour un comprimé terminé à | 20 mg<br>150 mg |
| (Détails de l'excipient : lactose, amidon, talc, stéarate de magnésium). | |

**Exemple 10 :**

On a préparé des comprimés répondant à la formulation suivante :

| | |
|---|---|
| - Produit de l'exemple 4<br>- Excipient pour un comprimé terminé à | 20 mg<br>150 mg |
| (Détails de l'excipient : lactose, amidon, talc, stéarate de magnésium). | |

**ACTIVITE PHARMACOLOGIOUE**

L'affinité des composés pour les récepteurs des benzodiazépines a été évaluée en utilisant un radioligand [$^3$H] flunitrazépam et la méthode de Squires et Braestrup (Nature, 1977, 266, 732) modifiée.

Les valeurs indiqués dans le tableau ci-après sont les concentrations nanomolaires du produit testé qui inhibent dans une proportion de 50% la liaison spécifique de 0,6 nanomoles de [$^3$H] flunitrazépam dans des préparations de membranes de cerveaux antérieurs de rats [($CI_{50}$) en nanomoles].

TABLEAU II

| Exemple | Liaison aux récepteurs $CI_{50}$ nm |
|---|---|
| 1 | 2,5 |
| 2 | 20 |
| 3 | 1000 |
| 4 | 3,2 |
| 5 | 158 |
| 6 | 79 |
| 7 | 6,3 |
| 8 | 60 |

**Revendications**

**Revendications pour les Etats contractants suivants : AT, BE, CH, DE, FR, IT, LI, LU, NL, SE**

**1.** Nouveaux imidazo [2,1-b] benzothiazoles répondant à la formule générale (I) :

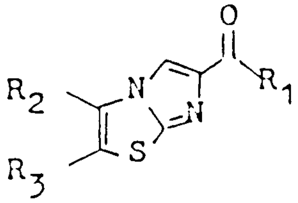

(I)

dans laquelle $R_1$ représente un groupement de formule (A) :

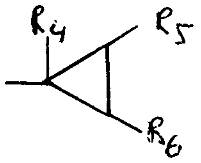

(A)

dans laquelle $R_4$ représente un atome d'hydrogène ou un radical alcoyle renfermant de 1 à 5 atomes de carbone, $R_5$ représente un atome d'hydrogène ou un radical alcoyle renfermant de 1 à 5 atomes de

carbone, $R_6$ représente un atome d'hydrogène, un radical alcoyle renfermant de 1 à 5 atomes de carbone, un radical phényle, un atome d'halogène, un radical alkoxycarbonyle renfermant de 2 à 5 atomes de carbone, un radical cyano, un radical amido, un radical mono ou dialcoylamido dont les groupements alcoyles renferment de 1 à 5 atomes de carbone ou $R_1$ représente un groupement de formule (B) :

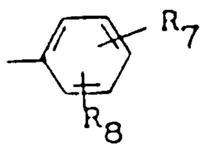

(B)

dans laquelle $R_7$ et $R_8$, identiques ou différents, représentent un atome d'hydrogène,un radical alcoyle, linéaire ou ramifié, renfermant de 1 à 5 atomes de carbone, $R_2$ et $R_3$ représentent un groupement :

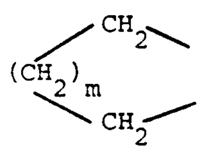

ou

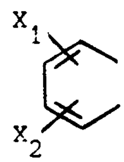

dans lequel m représente le nombre 1, 2 ou 3, $X_1$ et $X_2$, identiques ou différents, représentent un atome d'hydrogène, un radical alcoyle linéaire ou ramifié, renfermant de 2 à 5 atomes de carbone, un radical alkoxy, linéaire ou ramifié, renfermant de 1 à 5 atomes de carbone, un radical aralkoxy, un radical aryloxy, un atome d'halogène, un groupement nitrile ou azido, ou $X_1$ et $X_2$ forment ensemble un radical méthylènedioxy, ainsi que leurs sels d'addition avec les acides minéraux ou organiques.

**2.** Nouveaux imidazo [2,1-b] benzothiazoles tels que définis par la formule (I) de la revendication 1, caractérisés en ce que dans ladite formule (I) $R_1$ représente soit un groupement de formule (A) dans laquelle $R_4$, $R_5$ et $R_6$ représentent un atome d'hydrogène, soit un groupement de formule (B) dans laquelle $R_7$ et $R_8$ représentent un atome d'hydrogène et $R_2$ et $R_3$ ensemble représentent un groupement

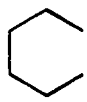

ou

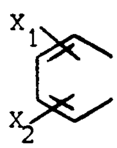

dans lequel $X_1$ et $X_2$, identiques ou différents, représentent un atome d'hydrogène, un radical méthyle, un radical méthoxy, un radical isopropoxy ou un radical benzyloxy, ainsi que leurs sels d'addition avec les acides minéraux ou organiques.

**3.** Nouveaux imidazo [2,1-b] benzothiazoles tels que définis à la revendication 1 ou 2, caractérisés en ce que dans ladite formule (I), $R_1$ représente un groupement de formule (A) dans laquelle $R_4$, $R_5$ et $R_6$ représentent un atome d'hydrogène et $R_2$ et $R_3$ ensemble représentent un groupement

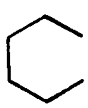

ou

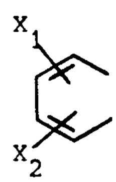

dans lequel $X_1$ représente un atome d'hydrogène et $X_2$ représente un radical méthoxy, un radical isopropoxy ou un radical benzyloxy, ainsi que leurs sels d'addition avec les acides minéraux ou organiques.

**4.** La (7-isopropoxyimidazo [2,1-b] benzothiazol-2-yl) cyclopropylméthanone et ses sels.

**5.** La (7-méthoxyimidazo [2,1-b] benzothiazol-2-yl) cyclopropylméthanone et ses sels.

**6.** La (7-benzyloxyimidazo [2,1-b] benzothiazol-2-yl) cyclopropylméthanone,

**7.** La (5,6,7,8-tétrahydroimidazo [2,1-b] benzothiazol-2-yl) cyclopropyl méthanone et ses sels.

**8.** Procédé de préparation des nouveaux imidazo [2,1-b] benzothiazoles tels que définis par la formule (I) de la revendication 1, répondant à la formule ($I_A$) :

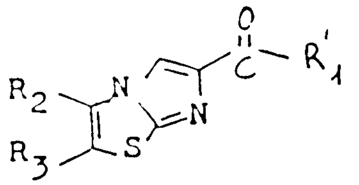

($I_A$)

dans laquelle $R_2$ et $R_3$ ont la signification déjà indiquée et $R'_1$ représente un groupement de formule (A) dans lequel $R_4$ représente un atome d'hydrogène et $R_5$ ou $R_6$ représentent un atome d'hydrogène ou un radical alcoxyle renfermant de 1 à 5 atomes de carbone ou $R'_1$ représente un groupement de formule (B) dans lequel $R_7$ et $R_8$ ont la signification indiquée, caractérisé en ce que soit l'on fait réagir un produit de formule (VIII) :

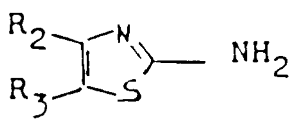

(VIII)

dans laquelle $R_2$ et $R_3$ ont la signification déjà indiquée avec du 3-bromo 1-hydroxypyran-2-one pour obtenir un produit de formule (VII) :

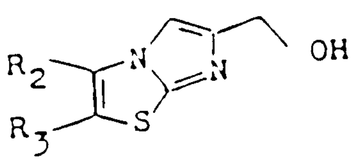

(VII)

dans laquelle $R_2$ et $R_3$ ont la signification déjà indiquée, soit l'on réduit un produit de formule (IX) :

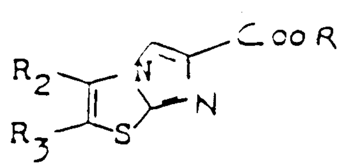

(IX)

dans laquelle $R_2$ et $R_3$ ont la signification déjà indiquée et R représente un radical alcoyle renfermant de 1 à 3 atomes de carbone, pour obtenir un produit de formule (VII) telle que définie ci-dessus, produit de formule (VII) que l'on soumet à un agent d'oxydation pour obtenir un produit de formule (V) :

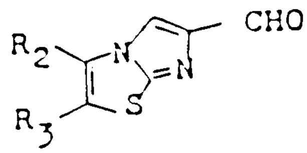

(V)

dans laquelle $R_2$ et $R_3$ ont la signification déjà indiquée que l'on fait réagir avec un produit de formule (VI) :

M-R'$_1$ (VI)

dans laquelle M représente un atome de métal alcalin lithium ou un reste - Mg Hal dans lequel Hal représente un atome de chlore, de brome ou d'iode et $R_1$ a la signification déjà indiquée pour obtenir un produit de formule (IV) :

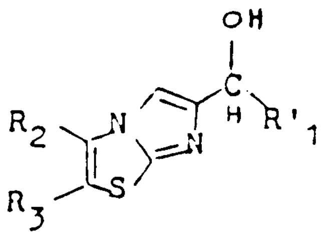

(IV)

dans laquelle R'$_1$, $R_2$ et $R_3$ ont la signification déjà indiquée, puis oxyde le produit de formule (IV) ainsi obtenu, pour obtenir le produit de formule ($I_A$) que l'on peut salifier le cas échéant.

**9.** Procédé de préparation selon la revendication 8, caractérisé en ce que :
- la réaction du produit de formule (V) avec le produit de formule (VI) est effectuée dans des conditions anhydres, dans un solvant organique tel que le tétrahydrofuranne,
- l'oxydation du produit de formule (IV) est effectuée avec le dioxyde de manganèse, l'acide nitrique, le chlorure ferrique ou l'oxyde de chrome en présence de pyridine ou encore par la méthode d'Oppenauer ou enfin par déshydrogénation en prsence d'un catalyseur à base de cuivre,
- l'oxydation du produit de formule (VII) peut avantageusement être effectuée au moyen de dioxyde de manganèse,
- la réduction du produit de formule (IX) peut avantageusement être effectuée au moyen du borohydrure de lithium.

**10.** Procédé de préparation des nouveaux imidazo [2,1-b] benzothiazoles tels que définis par la formule (I) de la revendication 1, répondant à la formule ($I_B$) :

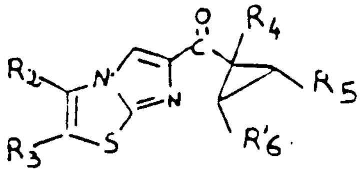

($I_B$)

dans laquelle $R_2$, $R_3$, $R_4$ et $R_5$ ont la signification déjà indiquée et R'$_6$ représente un atome d'hydrogène, un atome d'halogène, un radical alcoyle renfermant de 1 à 5 atomes de carbone, un radical alkoxycarbonyle renfermant de 2 à 5 atomes de carbone, un radical phényle, un radical cyano ou un groupement

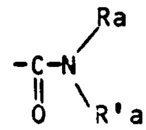

dans lequel Ra et R'a, identiques ou différents, représentent un atome d'hydrogène ou un radical alcoyle renfermant de 1 à 5 atomes de carbone, caractérisé en ce que l'on fait réagir un produit (V) telle que définie ci-dessus avec un produit de formule (XII) :

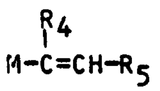

(XII)

dans laquelle M, $R_4$ et $R_5$ ont la signification déjà indiquée pour obtenir un produit de formule (XI) :

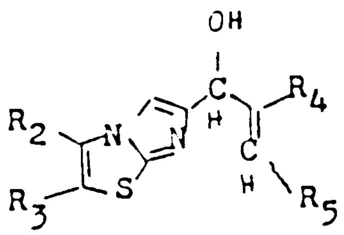

(XI)

dans laquelle $R_2$, $R_3$, $R_4$ et $R_5$ ont la signification déjà indiquée, pour oxyder le produit de formule (XI), pour obtenir un produit de formule (X) :

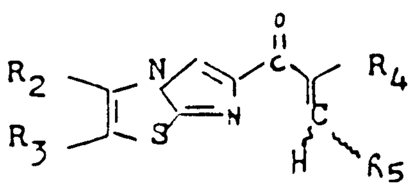

(X)

dans laquelle $R_2$, $R_3$, $R_4$ et $R_5$ ont la signification déjà indiquée, puis fait réagir ce dernier avec un agent de cyclisation approprié, pour obtenir un produit de formule ($I_B$) dans laquelle $R_2$, $R_3$, $R_4$ et $R_5$ ont la signification déjà indiquée et $R'_6$ représente un atome d'hydrogène, un atome d'halogène, un radical alcoyle renfermant de 1 à 5 atomes de carbone, un radical alcoxycarbonyle contenant de 2 à 5 atomes de carbone ou un radical phényle, puis saponifie le cas échéant le produit obtenu dans lequel $R'_6$ représente un radical alcoxycarbonyle pour obtenir un produit de formule (XIV) :

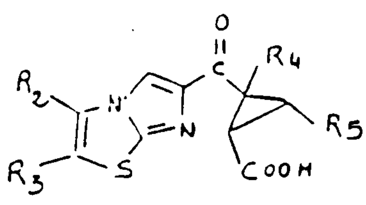

(XIV)

dans laquelle $R_2$, $R_3$, $R_4$ et $R_5$ ont la signification déjà indiquée, puis fait réagir ledit produit de formule (XIV) avec une amine de formule (XIII) :

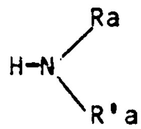

(XIII)

dans laquelle Ra et R'a ont la signification déjà indiquée pour obtenir le produit correspondant de formule ($I_B$) dans laquelle $R'_6$ représente un groupement

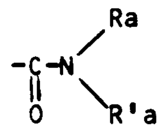

puis le cas échéant, déshydrate ce dernier dans lequel Ra et R'a représentent un atome d'hydrogène pour obtenir le produit correspondant de formule ($I_B$) dans laquelle $R'_6$ représente un radical cyano, et le cas échéant salifie les produits de formule ($I_B$) ainsi obtenus.

**11.** Procédé de préparation selon la revendication 10, caractérisé en ce que :

- la réaction du produit de formule (V) avec le produit de formule (XII) est effectuée dans des conditions anhydres, dans un solvant organique tel que le tétrahydrofuranne,
- l'oxydation du produit de formule (XI) est effectuée de préférence avec le dioxyde de manganèse, l'acide nitrique, le chlorure ferique ou l'oxyde de chrome, en présence de pyridine, ou encore par la méthode d'Oppenauer ou enfin par déshydrogénation en présence d'un catalyseur à base de cuivre,
- l'agent de cyclisation approprié est un réactif capable d'introduire le groupement CH-$R'_6$ au niveau de la double liaison,
- lorsque l'on désire obtenir un produit de formule ($I_B$) dans laquelle $R_4$ représente un radical alcoyle renfermant de 1 à 5 atomes de carbone, $R_5$ représente un atome d'hydrogène et $R'_6$ représente un atome d'hydrogène ou un radical alcoyle renfermant de 1 à 5 atomes de crbone, la cyclisation est avantageusement effectuée au moyen d'un iodure de trialkyloxosulphonium en opérant au sein d'un solvant organique tel que le diméthylformamide,
- lorsque l'on désire obtenir un produit de formule ($I_B$) dans laquelle $R_4$ représente un atome d'hydrogène, $R_5$ représente un radical alcoyle renfermant de 1 à 3 atomes de carbone et $R'_6$ représente un atome d'hydrogène ou un radical alcoyle renfermant de 1 à 5 atomes de carbone, la cyclisation est avantageusement effectuée au moyen d'un tétrafluoroborate de diméthylaminoalkylphényloxosulfonium en opérant au sein d'un solvant organique tel que le diméthylformamide,
- lorsque l'on désire obtenir un produit de formule ($I_B$) dans laquelle $R_4$ et $R_5$ représentent un atome d'hydrogène et $R'_6$ représente un groupement alcoxycarbonyle, le cyclisation est avantageusement effectuée au moyen d'un acétate d'alcoyle de diméthylsulfuranylidène ou d'un anion benzyl diméthylsulfonium en opérant au sein d'un solvant organique tel que le chloroforme,
- lorsque l'on désire obtenir un produit de formule ($I_B$) dans laquelle $R_4$ et $R_5$ représentent un atome d'hydrogène et $R'_6$ représente un atome d'halogène, la cyclisation est avantageusement effectuée au moyen d'un halogèno-méthylylidène de diméthylaminophénylsulfonium en opérant au sein d'un solvant organique tel que le diméthylformamide,
- la saponification du produit de formule ($I_B$) dans laquelle $R'_6$ représente un radical alkoxycarbonyle est effectuée de préférence au moyen d'un hydroxyde alcalin tel que l'hydroxyde de sodium,
- la réaction du produit de formule ( XIV) avec l'amine de formule (XIII) est effectuée de préférence au sein d'un solvant organique anhydre en présence de carbonyldiimidazole,
- la déshydratation du produit de formule ($I_B$) est effectuée de préférence au moyen d'un anhydride d'acide fort tel que l'anhydride d'acide trifluoroacétique au sein d'un solvant organique tel que le dichlorométhane.

**12.** Médicaments, caractérisés en ce qu'ils sont constitués par les nouveaux imidazo [2,1-b] benzothiazoles tels que définis par la formule (I) de la revendication 1, ainsi que par leurs sels pharmaceutiquement acceptables.

13. Médicaments, caractérisés en ce qu'ils sont constitués par les nouveaux imidazo [2,1-b] benzothiazoles tels que définis à l'une quelconque des revendications 2 à 8, ainsi que par leurs sels pharmaceutiquement acceptables.

14. Compositions pharmaceutiques, caractérisées en ce qu'elles renferment à titre de principe actif, l'un au moins des médicaments tels que définis à la revendication 12 ou 13.

15. A titre d'intermédiaires nécessaires à la préparation des produits de formule (I) telle que définie à la revendication 1,
   - les produits de formule (IV) :

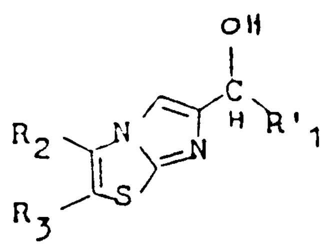

(IV)

dans laquelle $R'_1$, $R_2$ et $R_3$ ont la signification déjà indiquée ;
   - les produits de formule (XI) :

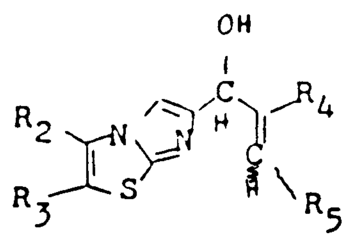

(XI)

dans laquelle $R_1$, $R_2$, $R_3$, $R_4$ et $R_5$ ont la signification déjà indiquée ;
   - les produits de formule (X) :

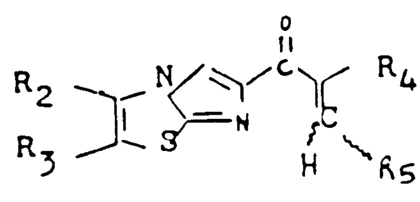

(X)

dans laquelle $R_2$, $R_3$, $R_4$ et $R_5$ on la signification déjà indiquée ;
   - les produits de formule (XIV) :

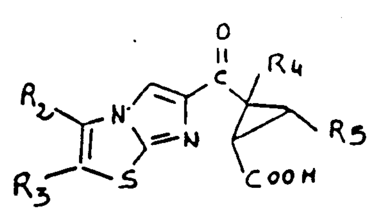

(XIV)

dans laquelle $R_2$, $R_3$, $R_4$ et $R_5$ ont la signification déjà indiquée.

**Revendications pour l'Etat contractant suivant : ES**

1. Procédé de préparation des nouveaux imidazo [2,1-b] benzothiazoles répondant à la formule générale (I) :

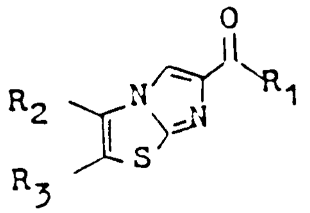

(I)

dans laquelle $R_1$ représente un groupement de formule (A) :

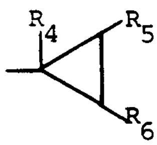

(A)

dans laquelle $R_4$ représente un atome d'hydrogène ou un radical alcoyle renfermant de 1 à 5 atomes de carbone, $R_5$ représente un atome d'hydrogène ou un radical alcoyle renfermant de 1 à 5 atomes de carbone, $R_6$ représente un atome d'hydrogène, un radical alcoyle renfermant de 1 à 5 atomes de carbone, un radical phényle, un atome d'halogène, un radical alkoxycarbonyle renfermant de 2 à 5 atomes de carbone, un radical cyano, un radical amido, un radical mono ou dialcoylamido dont les groupements alcoyles renferment de 1 à 5 atomes de carbone ou $R_1$ représente un groupement de formule (B) :

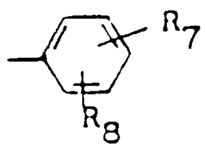

(B)

dans laquelle $R_7$ et $R_8$, identiques ou différents, représentent un atome d'hydrogène,un radical alcoyle, linéaire ou ramifié, renfermant de 1 à 5 atomes de carbone, $R_2$ et $R_3$ représentent un groupement :

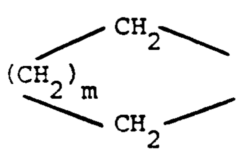

ou

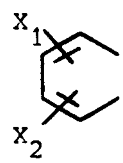

dans lequel m représente le nombre 1, 2 ou 3, $X_1$ et $X_2$, identiques ou différents, représentent un atome d'hydrogène, un radical alcoyle linéaire ou ramifié, renfermant de 2 à 5 atomes de carbone, un radical alkoxy, linéaire ou ramifié, renfermant de 1 à 5 atomes de carbone, un radical aralkoxy, un radical aryloxy, un atome d'halogène, un groupement nitrile ou azido, ou $X_1$ et $X_2$ forment ensemble un radical méthylènedioxy, ainsi que de leurs sels d'addition avec les acides minéraux ou organiques, caractérisé en ce que :

a) pour préparer des produits répondant à la formule ($I_A$) :

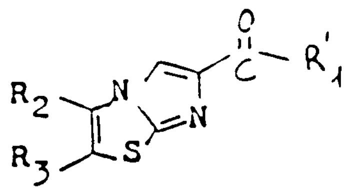

($I_A$)

dans laquelle $R_2$ et $R_3$ ont la signification déjà indiquée et $R'_1$ représente un groupement de formule (A) dans lequel $R_4$ représente un atome d'hydrogène et $R_5$ ou $R_6$ représentent un atome d'hydrogène ou un radical alcoxyle renfermant de 1 à 5 atomes de carbone ou $R'_1$ représente un groupement de formule (B) dans lequel $R_7$ et $R_8$ ont la signification indiquée, soit l'on fait réagir un produit de formule (VIII) :

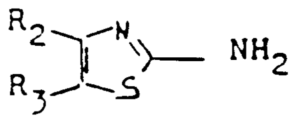

(VIII)

dans laquelle $R_2$ et $R_3$ ont la signification déjà indiquée avec du 3-bromo 1-hydroxypyran-2-one pour obtenir un produit de formule (VII) :

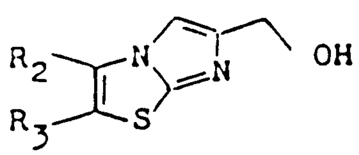

(VII)

dans laquelle $R_2$ et $R_3$ ont la signification déjà indiquée, soit l'on réduit un produit de formule (IX) :

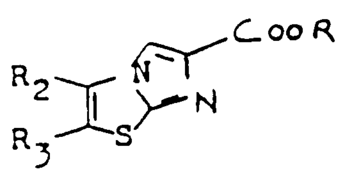

(IX)

dans laquelle $R_2$ et $R_3$ ont la signification déjà indiquée et R représente un radical alcoyle renfermant de 1 à 3 atomes de carbone, pour obtenir un produit de formule (VII) telle que définie ci-dessus, produit de formule (VII) que l'on soumet à un agent d'oxydation pour obtenir un produit de formule (V) :

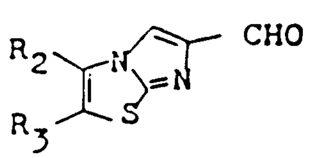

(V)

dans laquelle $R_2$ et $R_3$ ont la signification déjà indiquée que l'on fait réagir avec un produit de formule (VI) :

M-$R'_1$ (VI)

dans laquelle M représente un atome de métal alcalin lithium ou un reste - Mg Hal dans lequel Hal représente un atome de chlore, de brome ou d'iode et $R_1$ a la signification déjà indiquée pour obtenir un produit de formule (IV) :

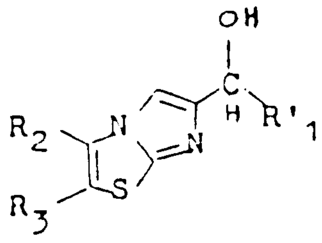

(IV)

dans laquelle $R'_1$, $R_2$ et $R_3$ ont la signification déjà indiquée, puis oxyde le produit de formule (IV) ainsi obtenu, pour obtenir le produit de formule ($I_A$) que l'on peut salifier le cas échéant ;
b) pour préparer des produits répondant à la formule ($I_B$) :

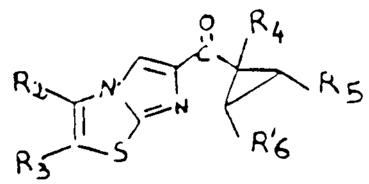

($I_B$)

dans laquelle $R_2$, $R_3$, $R_4$ et $R_5$ ont la signification déjà indiquée et $R'_6$ représente un atome d'hydrogène, un atome d'halogène, un radical alcoyle renfermant de 1 à 5 atomes de carbone, un radical alkoxycarbonyle renfermant de 2 à 5 atomes de carbone et un groupement phényl, un radical cyano ou un groupement

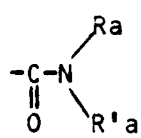

dans lequel Ra et R'a, identiques ou différents, représentent un atome d'hydrogène ou un radical alcoyle renfermant de 1 à 5 atomes de carbone, l'on fait réagir un produit (V) telle que définie ci-dessus avec un produit de formule (XII) :

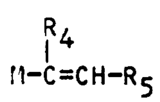

(XII)

dans laquelle M, $R_4$ et $R_5$ ont la signification déjà indiquée pour obtenir un produit de formule (XI) :

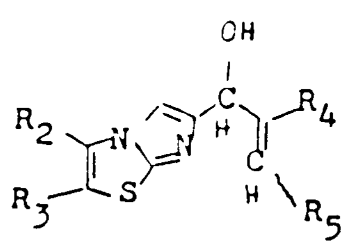

(XI)

dans laquelle $R_2$, $R_3$, $R_4$ et $R_5$ ont la signification déjà indiquée, pour oxyder le produit de formule (XI), pour obtenir un produit de formule (X) :

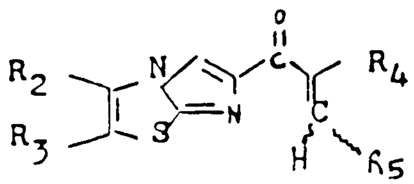

(X)

dans laquelle $R_2$, $R_3$, $R_4$ et $R_5$ ont la signification déjà indiquée, puis fait réagir ce dernier avec un agent de cyclisation approprié, pour obtenir un produit de formule ($I_B$) dans laquelle $R_2$, $R_3$, $R_4$ et $R_5$ ont la signification déjà indiquée et $R'_6$ représente un atome d'hydrogène, un atome d'halogène, un radical alcoyle renfermant de 1 à 5 atomes de carbone, un radical alcoxycarbonyle contenant de 2 à 5 atomes de carbone ou un radical phényle, puis saponifie le cas échéant le produit obtenu dans lequel $R'_6$ représente un radical alcoxycarbonyle pour obtenir un produit de formule (XIV) :

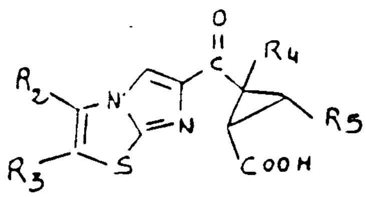

(XIV)

dans laquelle $R_2$, $R_3$, $R_4$ et $R_5$ ont la signification déjà indiquée, puis fait réagir ledit produit de formule (XIV) avec une amine de formule (XIII) :

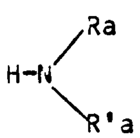

(XIII)

dans laquelle Ra et R'a ont la signification déjà indiquée pour obtenir le produit correspondant de formule ($I_B$) dans laquelle $R'_6$ représente un groupement

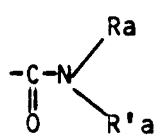

puis le cas échéant, déshydrate ce dernier dans lequel Ra et R'a représentent un atome d'hydrogène pour obtenir le produit correspondant de formule ($I_B$) dans laquelle $R'_6$ représente un radical cyano, et le cas échéant salifie les produits de formule ($I_B$) ainsi obtenus.

2. Procédé selon la revendication 1 pour la préparation de produits répondant à la formule (I) dans laquelle $R_1$ représente soit un groupement (A) dans laquelle $R_4$, $R_5$ et $R_6$ représentent un atome d'hydrogène, soit un groupement de formule (B) dans laquelle $R_7$ et $R_8$ représentent un atome d'hydrogène et $R_2$ et $R_3$ ensemble représentent un groupe

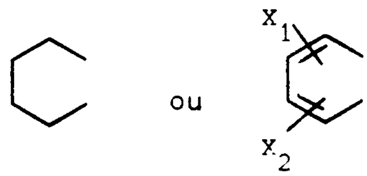

dans lequel $X_1$ et $X_2$, identiques ou différents, représentent un atome d'hydrogène, un radical méthyle, un radical méthoxy, un radical isopropoxy ou un radical benzyloxy, ainsi que de leurs sels d'addition avec les acides minéraux ou organiques, caractérisé en ce que l'on utilise au départ un composé de formule (VIII) et (IX) dans laquelle $R_2$ et $R_3$ ont la signification indiquée ci-dessus et un composé de formule (VI) dans laquelle $R'_1$ représente un groupement de formule (A) dans laquelle $R_4$, $R_5$ et $R_6$ représentent un atome d'hydrogène ou un groupement de formule (B) dans laquelle $R_7$ et $R_8$ représentent un atome d'hydrogène ou bien un composé de formule (XII) dans laquelle $R_4$ et $R_5$ représentent un atome d'hydrogène et un réactif de cyclisation approprié.

**3.** Procédé selon la revendication 1 ou 2 pour la préparation des produits répondant à la formule (I) dans laquelle $R_1$ représente un groupement de formule (A) dans laquelle $R_4$, $R_5$ et $R_6$ représentent un atome d'hydrogène et $R_2$ et $R_3$ ensemble représentent un groupement

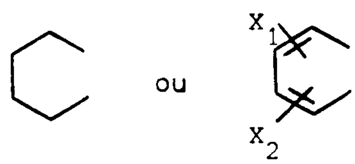

dans lequel $X_1$ représente un atone d'hydrogène et $X_2$ représente un radical méthoxy, un radical isopropoxy ou un radical benzyloxy, ainsi que de leurs sels d'addition avec les acides minéraux ou organiques, caractérisé en ce que l'on utilise au départ un composé de formule (VIII) ou (IX) dans laquelle $R_2$ et $R_3$ ont la signification indiquée ci-dessus et un composé de formule (VI) dans laquelle $R'_1$ représente un groupement de formule (A) dans laquelle $R_4$, $R_5$ et $R_6$ représentent un atome d'hydrogène ou bien un composé de formule (XII) dans laquelle $R_4$ et $R_5$ représentent un atome d'hydrogène et un agent de cyclisation approprié.

**Revendications pour l'Etat contractant suivant : GR**

**1.** Procédé de préparation des nouveaux imidazo [2,1-b] benzothiazoles répondant à la formule générale (I) :

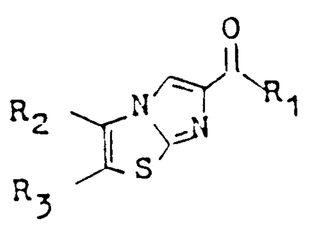

(I)

dans laquelle $R_1$ représente un groupement de formule (A) :

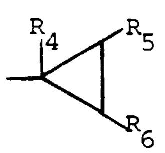

(A)

dans laquelle $R_4$ représente un atome d'hydrogène ou un radical alcoyle renfermant de 1 à 5 atomes de carbone, $R_5$ représente un atome d'hydrogène ou un radical alcoyle renfermant de 1 à 5 atomes de

carbone, $R_6$ représente un atome d'hydrogène, un radical alcoyle renfermant de 1 à 5 atomes de carbone, un radical phényle, un atome d'halogène, un radical alkoxycarbonyle renfermant de 2 à 5 atomes de carbone, un radical cyano, un radical amido, un radical mono ou dialcoylamido dont les groupements alcoyles renferment de 1 à 5 atomes de carbone ou $R_1$ représente un groupement de formule (B) :

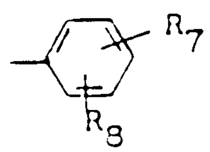

(B)

dans laquelle $R_7$ et $R_8$, identiques ou différents, représentent un atome d'hydrogène,un radical alcoyle, linéaire ou ramifié, renfermant de 1 à 5 atomes de carbone, $R_2$ et $R_3$ représentent un groupement :

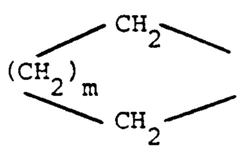

ou

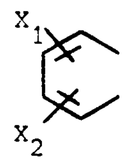

dans lequel m représente le nombre 1, 2 ou 3, $X_1$ et $X_2$, identiques ou différents, représentent un atome d'hydrogène, un radical alcoyle linéaire ou ramifié, renfermant de 2 à 5 atomes de carbone, un radical alkoxy, linéaire ou ramifié, renfermant de 1 à 5 atomes de carbone, un radical aralkoxy, un radical aryloxy, un atome d'halogène, un groupement nitrile ou azido, ou $X_1$ et $X_2$ forment ensemble un radical méthylènedioxy, ainsi que de leurs sels d'addition avec les acides minéraux ou organiques, caractérisé en ce que :

a) pour préparer des produits répondant à la formule ($I_A$) :

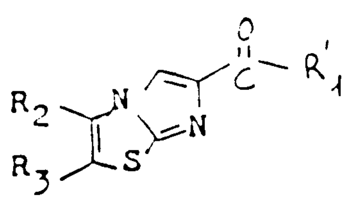

($I_A$)

dans laquelle $R_2$ et $R_3$ ont la signification déjà indiquée et $R'_1$ représente un groupement de formule (A) dans lequel $R_4$ représente un atome d'hydrogène et $R_5$ ou $R_6$ représentent un atome d'hydrogène ou un radical alcoxyle renfermant de 1 à 5 atomes de carbone ou $R'_1$ représente un groupement de formule (B) dans lequel $R_7$ et $R_8$ ont la signification indiquée, soit l'on fait réagir un produit de formule (VIII) :

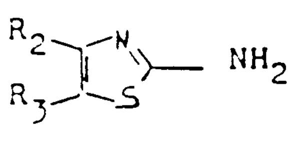

(VIII)

dans laquelle $R_2$ et $R_3$ ont la signification déjà indiquée avec du 3-bromo 1-hydroxypyran-2-one pour obtenir un produit de formule (VII) :

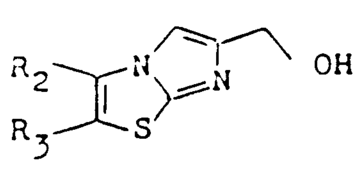

(VII)

dans laquelle $R_2$ et $R_3$ ont la signification déjà indiquée, soit l'on réduit un produit de formule (IX) :

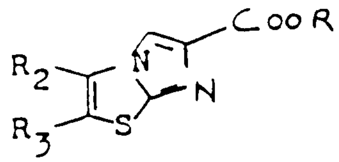

(IX)

dans laquelle $R_2$ et $R_3$ ont la signification déjà indiquée et R représente un radical alcoyle renfermant de 1 à 3 atomes de carbone, pour obtenir un produit de formule (VII) telle que définie ci-dessus, produit de formule (VII) que l'on soumet à un agent d'oxydation pour obtenir un produit de formule (V) :

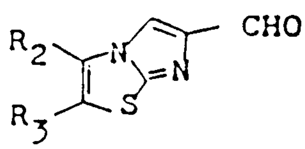

(V)

dans laquelle $R_2$ et $R_3$ ont la signification déjà indiquée que l'on fait réagir avec un produit de formule (VI) :

M-R'$_1$ (VI)

dans laquelle M représente un atome de métal alcalin lithium ou un reste - Mg Hal dans lequel Hal représente un atome de chlore, de brome ou d'iode et $R_1$ a la signification déjà indiquée pour obtenir un produit de formule (IV) :

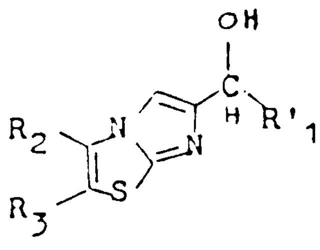

(IV)

dans laquelle $R'_1$, $R_2$ et $R_3$ ont la signification déjà indiquée, puis oxyde le produit de formule (IV) ainsi obtenu, pour obtenir le produit de formule ($I_A$) que l'on peut salifier le cas échéant ;
b) pour prépa

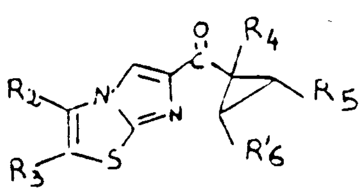

($I_B$)

rer des produits répondant à la formule ($I_B$) : dans laquelle $R_2$, $R_3$, $R_4$ et $R_5$ ont la signification déjà indiquée et $R'_6$ représente un atome d'hydrogène, un atome d'halogène, un radical alcoyle renfermant de 1 à 5 atomes de carbone, un radical alkoxycarbonyle renfermant de 2 à 5 atomes de carbone et un groupement phényl, un radical cyano ou un groupement

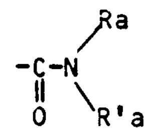

dans lequel Ra et R'a, identiques ou différents, représentent un atome d'hydrogène ou un radical alcoyle renfermant de 1 à 5 atomes de carbone, l'on fait réagir un produit (V) telle que définie ci-dessus avec un produit de formule (XII) :

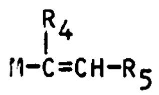

(XII)

dans laquelle M, $R_4$ et $R_5$ ont la signification déjà indiquée pour obtenir un produit de formule (XI) :

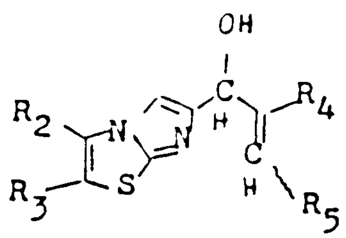

(XI)

dans laquelle $R_2$, $R_3$, $R_4$ et $R_5$ ont la signification déjà indiquée, pour oxyder le produit de formule (XI), pour obtenir un produit de formule (X) :

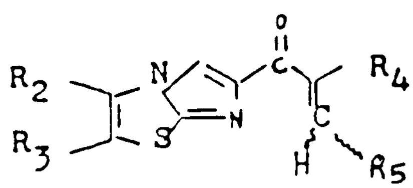

(X)

dans laquelle $R_2$, $R_3$, $R_4$ et $R_5$ ont la signification déjà indiquée, puis fait réagir ce dernier avec un agent de cyclisation approprié, pour obtenir un produit de formule ($I_B$) dans laquelle $R_2$, $R_3$, $R_4$ et $R_5$ ont la signification déjà indiquée et $R'_6$ représente un atome d'hydrogène, un atome d'halogène, un radical alcoyle renfermant de 1 à 5 atomes de carbone, un radical alcoxycarbonyle contenant de 2 à 5 atomes de carbone ou un radical phényle, puis saponifie le cas échéant le produit obtenu dans lequel $R'_6$ représente un radical alcoxycarbonyle pour obtenir un produit de formule (XIV) :

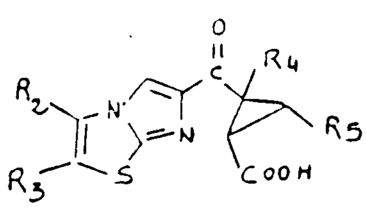

(XIV)

dans laquelle $R_2$, $R_3$, $R_4$ et $R_5$ ont la signification déjà indiquée, puis fait réagir ledit produit ce formule (XIV) avec une amine de formule (XIII) :

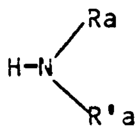

(XIII)

dans laquelle Ra et R'a ont la signification déjà indiquée pour obtenir le produit correspondant de formule ($I_B$) dans laquelle $R'_6$ représente un groupement

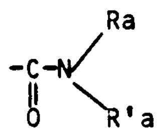

puis le cas échéant, déshydrate ce dernier dans lequel Ra et R'a représentent un atome d'hydrogène pour obtenir le produit correspondant de formule ($I_B$) dans laquelle $R'_6$ représente un radical cyano, et le cas échéant salifie les produits de formule ($I_B$) ainsi obtenus.

**2.** Procédé selon la revendication 1 pour la préparation de produits répondant à la formule (I) dans laquelle $R_1$ représente soit un groupement (A) dans laquelle $R_4$, $R_5$ et $R_6$ représentent un atome d'hydrogène, soit un groupement de formule (B) dans laquelle $R_7$ et $R_8$ représentent un atome d'hydrogène et $R_2$ et $R_3$ ensemble représentent un groupe

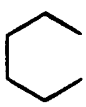

ou

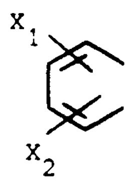

dans lequel $X_1$ et $X_2$, identiques ou différents, représentent un atome d'hydrogène, un radical méthyle, un radical méthoxy, un radical isopropoxy ou un radical benzyloxy, ainsi que de leurs sels d'addition avec les acides minéraux ou organiques, caractérisé en ce que l'on utilise au départ un composé de formule (VIII) et (IX) dans laquelle $R_2$ et $R_3$ ont la signification indiquée ci-dessus et un composé de formule (VI) dans laquelle $R'_1$ représente un groupement de formule (A) dans laquelle $R_4$, $R_5$ et $R_6$ représentent un atome d'hydrogène ou un groupement de formule (B) dans laquelle $R_7$ et $R_8$ représentent un atome d'hydrogène ou bien un composé de formule (XII) dans laquelle $R_4$ et $R_5$ représentent un atome d'hydrogène et un réactif de cyclisation approprié.

**3.** Procédé selon la revendication 1 ou 2 pour la préparation des produits répondant à la formule (I) dans laquelle $R_1$ représente un groupement de formule (A) dans laquelle $R_4$, $R_5$ et $R_6$ représentent un atome d'hydrogène et $R_2$ et $R_3$ ensemble représentent un groupement

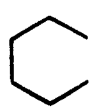

ou

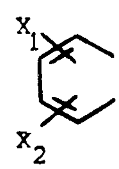

dans lequel $X_1$ représente un atome d'hydrogène et $X_2$ représente un radical méthoxy, un radical isopropoxy ou un radical benzyloxy, ainsi que de leurs sels d'addition avec les acides minéraux ou organiques, caractérisé en ce que l'on utilise au départ un composé de formule (VIII) ou (IX) dans laquelle $R_2$ et $R_3$ ont la signification indiquée ci-dessus et un composé de formule (VI) dans laquelle $R'_1$ représente un groupement de formule (A) dans laquelle $R_4$, $R_5$ et $R_6$ représentent un atome d'hydrogène ou bien un composé de formule (XII) dans laquelle $R_4$ et $R_5$ représentent un atome d'hydrogène et un agent de cyclisation approprié.

**4.** A titre de produits industriels nouveaux, les produits de formule (IV), (XI), (X) et (XIV) tels que définis à la revendication 1.

**5.** Procédé de préparation de compositions pharmaceutiques, caractérisé en ce que l'on met à titre de principe actif, l'un au moins des composés de formule (I) tels que définis à la revendication 1 ou l'un au moins de leurs sels d'addition avec les acides pharmaceutiquement acceptables sous une forme destinée à cet usage.

**Claims**

**Claims for the following Contracting States : AT, BE, CH, DE, FR, IT, LI, LU, NL, SE**

**1.** New imidazo [2,1-b] benzothiazoles corresponding to general formula (I):

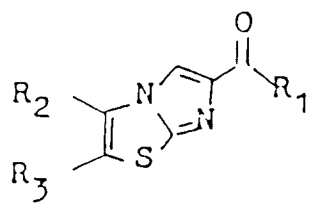

(I)

in which $R_1$ represents a group of formula (A):

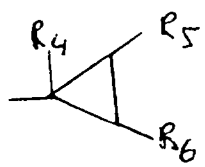

(A)

in which $R_4$ represents a hydrogen atom or an alkyl radical containing 1 to 5 carbon atoms, $R_5$ represents a hydrogen atom or an alkyl radical containing 1 to 5 carbon atoms, $R_6$ represents a hydrogen atom, an alkyl radical containing 1 to 5 carbon atoms, a phenyl radical, a halogen atom, an alkoxycarbonyl radical containing 2 to 5 carbon atoms, a cyano radical, an amido radical, a mono- or dialkylamido radical the alkyl groups of which contain 1 to 5 carbon atoms or $R_1$ represents a group of formula (B):

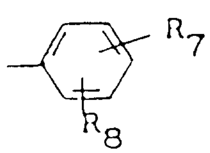

(B)

in which $R_7$ and $R_8$, identical or different, represent a hydrogen atom, a linear or branched alkyl radical containing 1 to 5 carbon atoms, $R_2$ and $R_3$ represent a group:

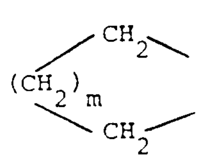

or

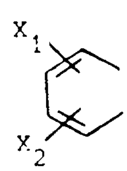

in which m represents the number 1, 2 or 3, $X_1$ and $X_2$, identical or different, represent a hydrogen

atom, a linear or branched alkyl radical containing 2 to 5 carbon atoms, a linear or branched alkoxy radical containing 1 to 5 carbon atoms, an aralkoxy radical, an aryloxy radical, a halogen atom, a nitrile or azido group, or $X_1$ and $X_2$ together form a methylenedioxy radical, as well as their addition salts with mineral or organic acids.

**2.** New imidazo [2,1-b] benzothiazoles as defined by formula (I) of claim 1, characterized in that in the said formula (I) $R_1$ represents either a group of formula (A) in which $R_4$, $R_5$ and $R_6$ represent a hydrogen atom, or a group of formula (B) in which $R_7$ and $R_8$ represent a hydrogen atom and $R_2$ and $R_3$ together represent a group:

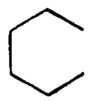

or

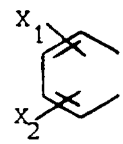

in which $X_1$ and $X_2$, identical or different, represent a hydrogen atom, a methyl radical, a methoxy radical, an isopropoxy radical or a benzyloxy radical, as well as their addition salts with mineral or organic acids.

**3.** New imidazo [2,1-b] benzothiazoles as defined in claim 1 or 2, characterized in that in the said formula (I), $R_1$ represents a group of formula (A) in which $R_4$, $R_5$ and $R_6$ represent a hydrogen atom and $R_2$ and $R_3$ together represent a group:

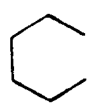

or

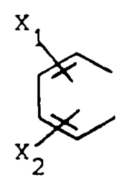

in which $X_1$ represents a hydrogen atom and $X_2$ represents a methoxy radical, an isopropoxy radical or a benzyloxy radical, as well as their addition salts with mineral or organic acids.

**4.** (7-isopropoxyimidazo [2,1-b] benzothiazol-2-yl) cyclopropylmethanone and its salts.

**5.** (7-methoxyimidazo [2,1-b] benzothiazol-2-yl) cyclopropylmethanone and its salts.

**6.** (7-benzyloxyimidazo [2,1-b] benzothiazol-2-yl) cyclopropylmethanone.

**7.** (5,6,7,8-tetrahydroimidazo [2,1-b] benzothiazol-2-yl) cyclopropyl methanone and its salts.

**8.** Preparation process for new imidazo [2,1-b] benzothiazoles as defined by formula (I) of claim 1, corresponding to formula ($I_A$):

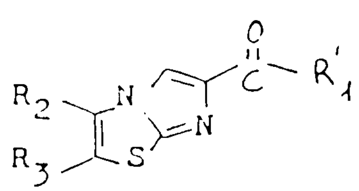

($I_A$)

in which $R_2$ and $R_3$ have the meaning already indicated and $R'_1$ represents a group of formula (A) in which $R_4$ represents a hydrogen atom and $R_5$ or $R_6$ represent a hydrogen atom or an alkoxyl radical containing 1 to 5 carbon atoms or $R'_1$ represents a group of formula (B) in which $R_7$ and $R_8$ have the

meaning indicated, characterized in that either a product of formula (VIII):

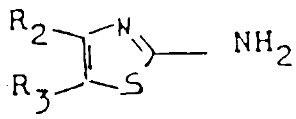

(VIII)

in which $R_2$ and $R_3$ have the meaning already indicated, is reacted with 3-bromo 1-hydroxypran-2-one in order to obtain a product of formula (VII):

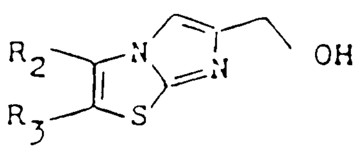

(VII)

in which $R_2$ and $R_3$ have the meaning already indicated, or a product of formula (IX):

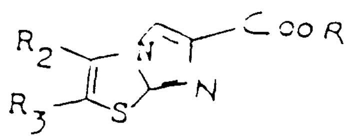

(IX)

in which $R_2$ and $R_3$ have the meaning already indicated and R represents an alkyl radical containing 1 to 3 carbon atoms, is reduced in order to obtain a product of formula (VII) as defined above, which product of formula (VII) is subjected to an oxidizing agent in order to obtain a product of formula (V):

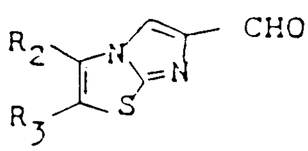

(V)

in which $R_2$ and $R_3$ have the meaning already indicated, which is reacted with a product of formula (VI):

$M\text{-}R'_1$ (VI)

in which M represents a lithium alkali metal atom or a - Mg Hal remainder in which Hal represents a chlorine, bromine or iodine atom and $R_1$ has the meaning already indicated, in order to obtain a product of formula (IV):

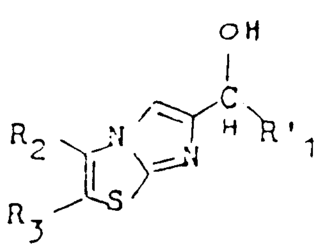

(IV)

in which $R'_1$, $R_2$ and $R_3$ have the meaning already indicated, then the product of formula (IV) thus obtained is oxidized in order to obtain the product of formula ($I_A$) which can be salified if appropriate.

**9.** Preparation process according to claim 8, characterized in that:

- the reaction of the product of formula (V) with the product of formula (VI) is carried out in anhydrous conditions, in an organic solvent such as tetrahydrofuran,
- the oxidation of the product of formula (IV) is carried out with manganese dioxide, nitric acid, ferric chloride or chromium oxide in the presence of pyridine or also by the Oppenauer method or finally by dehydrogenation in the presence of a copper-based catalyst,
- the oxidation of the product of formula (VII) can advantageously be carried out using manganese dioxide,
- the reduction of the product of formula (IX) can advantageously be carried out using lithium borohydride.

**10.** Preparation process for new imidazo [2,1-b] benzothiazoles as defined by formula (I) of claim 1, corresponding to formula ($I_B$):

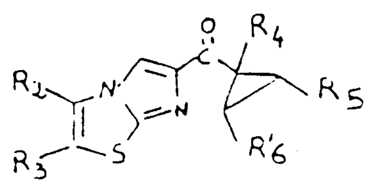

($I_B$)

in which $R_2$, $R_3$, $R_4$ and $R_5$ have the meaning already indicated and $R'_6$ represents a hydrogen atom, a halogen atom, an alkyl radical containing 1 to 5 carbon atoms, an alkoxycarbonyl radical containing 2 to 5 carbon atoms, a phenyl radical, a cyano radical or a

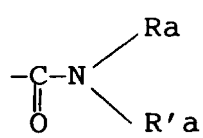

radical in which Ra and R'a, identical or different, represent a hydrogen atom or an alkyl radical containing 1 to 5 carbon atoms, characterized in that a product (V) as defined above is reacted with a product of formula (XII):

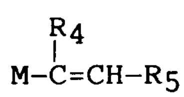

(XII)

in which M, $R_4$ and $R_5$ have the meaning already indicated, in order to obtain a product of formula (XI):

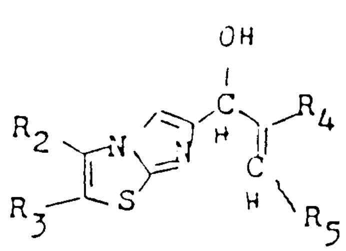

(XI)

in which $R_2$, $R_3$, $R_4$ and $R_5$ have the meaning already indicated, to oxidize the product of formula (XI), in order to obtain a product of formula (X):

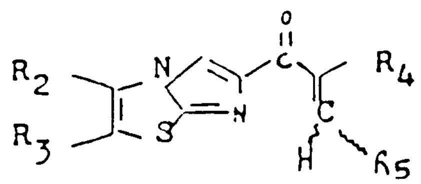

(X)

in which $R_2$, $R_3$, $R_4$ and $R_5$ have the meaning already indicated, then the latter is reacted with an appropriate cyclization agent, in order to obtain a product of formula ($I_B$) in which $R_2$, $R_3$, $R_4$ and $R_5$ have the meaning already indicated and $R'_6$ represents a hydrogen atom, a halogen atom, an alkyl radical containing 1 to 5 carbon atoms, an alkoxycarbonyl radical containing 2 to 5 carbon atoms or a phenyl radical, then if appropriate the product obtained in which $R'_6$ represents an alkoxycarbonyl radical is saponified in order to obtain a product of formula (XIV):

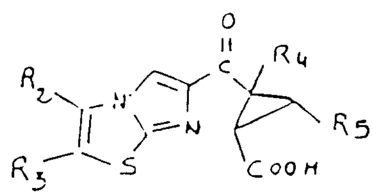

(XIV)

in which $R_2$, $R_3$, $R_4$ and $R_5$ have the meaning already indicated, then the said product of formula (XIV) is reacted with an amine of formula (XIII):

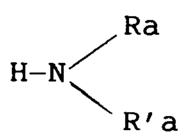

(XIII)

in which Ra and R'a have the meaning already indicated in order to obtain the corresponding product of formula ($I_B$) in which $R'_6$ represents a

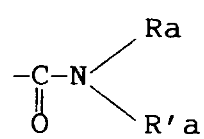

group, then if appropriate, the latter in which Ra and R'a represent a hydrogen atom is dehydrated in order to obtain the corresponding product of formula ($I_B$) in which $R'_6$ represents a cyano radical, and if appropriate the products of formula ($I_B$) thus obtained are salified.

**11.** Preparation process according to claim 10, characterized in that:

- the reaction of the product of formula (V) with the product of formula (XII) is carried out in anhydrous conditions, in an organic solvent such as tetrahydrofuran,
- the oxidation of the product of formula (XI) is preferably carried out with manganese dioxide, nitric acid, ferric chloride or chromium oxide, in the presence of pyridine, or also by the Oppenauer method or finally by dehydrogenation in the presence of a copper-based catalyst,
- the appropriate cyclization agent is a reagent capable of introducing the CH-$R'_6$ group at the level of the double bond,
- when it is desired to obtain a product of formula ($I_B$) in which $R_4$ represents an alkyl radical containing 1 to 5 carbon atoms, $R_5$ represents a hydrogen atom and $R'_6$ represents a hydrogen atom or an alkyl radical containing 1 to 5 carbon atoms, the cyclization is advantageously carried out using a trialkyloxosulphonium iodide operating in an organic solvent such as dimethyl

formamide,

- when it is desired to obtain a product of formula ($I_B$) in which $R_4$ represents a hydrogen atom, $R_5$ represents an alkyl radical containing 1 to 3 carbon atoms and $R'_6$ represents a hydrogen atom or an alkyl radical containing 1 to 5 carbon atoms, the cyclization is advantageously carried out using a dimethylaminoalkylphenyloxosulphonium tetrafluoroborate operating in an organic solvent such as dimethylformamide,
- when it is desired to obtain a product of formula ($I_B$) in which $R_4$ and $R_5$ represent a hydrogen atom and $R'_6$ represents an alkoxycarbonyl group, the cyclization is advantageously carried out using a dimethylsulphuranylidene alkyl acetate or a benzyl dimethylsulphonium anion operating in an organic solvent such as chloroform,
- when it is desired to obtain a product of formula ($I_B$) in which $R_4$ and $R_5$ represent a hydrogen atom and $R'_6$ represents a halogen atom, the cyclization is advantageously carried out using a dimethylaminophenylsulphonium halogeno-methylylidene operating in an organic solvent such as dimethylformamide,
- the saponification of the product of formula ($I_B$) in which $R'_6$ represents an alkoxycarbonyl radical is preferably carried out using an alkaline hydroxide such as sodium hydroxide,
- the reaction of the product of formula (XIV) with the amine of formula (XIII) is preferably carried out in an anhydrous organic solvent in the presence of carbonyldiimidazole,
- the dehydration of the product of formula ($I_B$) is preferably carried out using a strong acid anhydride such as trifluoroacetic acid anhydride in an organic solvent such as dichloromethane.

**12.** Medicaments, characterized in that they are constituted by the new imidazo [2,1-b] benzothiazoles as defined by formula (I) of claim 1, as well as by their pharmaceutically acceptable salts.

**13.** Medicaments, characterized in that they are constituted by the new imidazo [2,1-b] benzothiazoles as defined in any one of claims 2 to 8, as well as by their pharmaceutically acceptable salts.

**14.** Pharmaceutical compositions, characterized in that they contain as active ingredient at least one of the medicaments as defined in claim 12 or 13.

**15.** As intermediate products necessary for the preparation of products of formula (I) as defined in claim 1,

- the products of formula (IV):

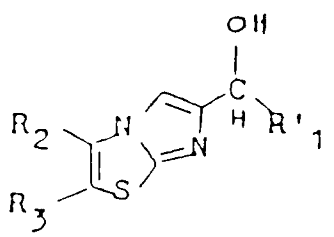

(IV)

in which $R'_1$, $R_2$ and $R_3$ have the meaning already indicated;

- the products of formula (XI):

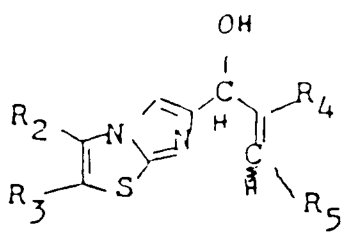

(XI)

in which $R_1$, $R_2$, $R_3$, $R_4$ and $R_5$ have the meaning already indicated;

- the products of formula (X):

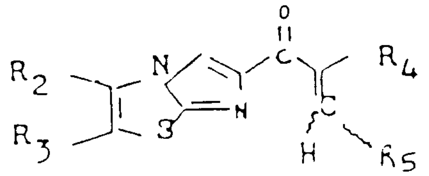

(X)

in which $R_2$, $R_3$, $R_4$ and $R_5$ have the meaning already indicated;

- the products of formula (XIV):

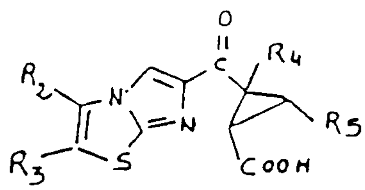

(XIV)

in which $R_2$, $R_3$, $R_4$ and $R_5$ have the meaning already indicated.

**Claims for the following Contracting State : ES**

**1.** Preparation process for new imidazo [2,1-b] benzothiazoles corresponding to general formula (I):

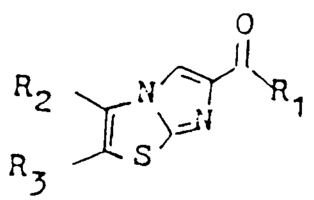

(I)

in which $R_1$ represents a group of formula (A):

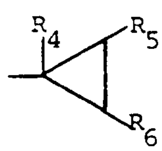

(A)

in which $R_4$ represents a hydrogen atom or an alkyl radical containing 1 to 5 carbon atoms, $R_5$ represents a hydrogen atom or an alkyl radical containing 1 to 5 carbon atoms, $R_6$ represents a hydrogen atom, an alkyl radical containing 1 to 5 carbon atoms, a phenyl radical, a halogen atom, an alkoxycarbonyl radical containing 2 to 5 carbon atoms, a cyano radical, an amido radical, a mono- or dialkylamido radical the alkyl groups of which contain 1 to 5 carbon atoms or $R_1$ represents a group of formula (B):

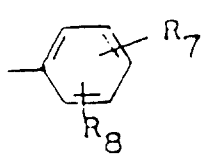

(B)

in which $R_7$ and $R_8$, identical or different, represent a hydrogen atom, a linear or branched alkyl radical containing 1 to 5 carbon atoms, $R_2$ and $R_3$ represent a group:

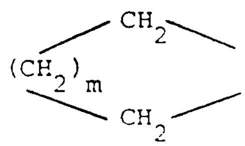

or

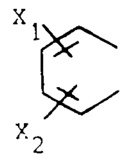

in which m represents the number 1, 2 or 3, $X_1$ and $X_2$, identical or different, represent a hydrogen atom, a linear or branched alkyl radical containing 2 to 5 carbon atoms, a linear or branched alkoxy radical containing 1 to 5 carbon atoms, an aralkoxy radical, an aryloxy radical, a halogen atom, a nitrile or azido group, or $X_1$ and $X_2$ together form a methylenedioxy radical, as well as their addition salts with mineral or organic acids, characterized in that: a) in order to prepare products corresponding to formula ($I_A$):

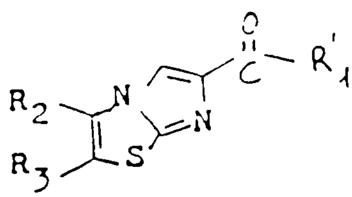

($I_A$)

in which $R_2$ and $R_3$ have the meaning already indicated and $R'_1$ represents a group of formula (A) in which $R_4$ represents a hydrogen atom and $R_5$ or $R_6$ represent a hydrogen atom or an alkoxyl radical containing 1 to 5 carbon atoms or $R'_1$ represents a group of formula (B) in which $R_7$ and $R_8$ have the meaning indicated, either a product of formula (VIII):

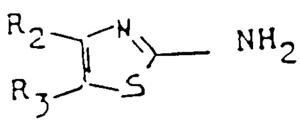

(VIII)

in which $R_2$ and $R_3$ have the meaning already indicated, is reacted with 3-bromo 1-hydroxypran-2-one in order to obtain a product of formula (VII):

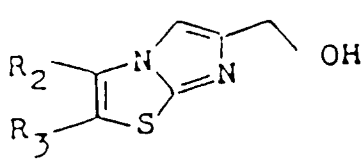

(VII)

in which $R_2$ and $R_3$ have the meaning already indicated, or a product of formula (IX):

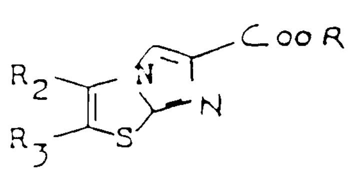

(IX)

in which $R_2$ and $R_3$ have the meaning already indicated and R represents an alkyl radical containing 1 to 3 carbon atoms, is reduced in order to obtain a product of formula (VII) as defined above, which product of formula (VII) is subjected to an oxidizing agent in order to obtain a product of formula (V):

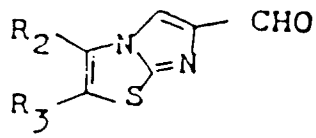

(V)

in which $R_2$ and $R_3$ have the meaning already indicated, which is reacted with a product of formula (VI):

M-R'$_1$ (VI)

in which M represents a lithium alkali metal atom or a - Mg Hal remainder in which Hal represents a chlorine, bromine or iodine atom and $R_1$ has the meaning already indicated, in order to obtain a product of formula (IV):

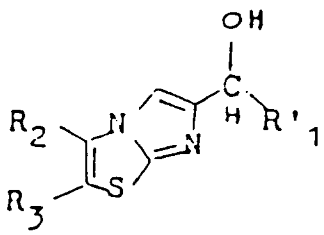

(IV)

in which R'$_1$, $R_2$ and $R_3$ have the meaning already indicated, then the product of formula (IV) thus obtained is oxidized in order to obtain the product of formula ($I_A$) which can be salified if appropriate; b) in order to prepare products corresponding to formula ($I_B$):

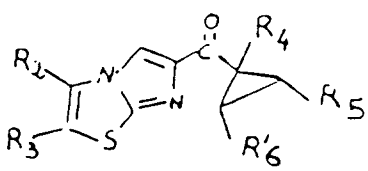

($I_B$)

in which $R_2$, $R_3$, $R_4$ and $R_5$ have the meaning already indicated and R'$_6$ represents a hydrogen atom, a halogen atom, an alkyl radical containing 1 to 5 carbon atoms, an alkoxycarbonyl radical containing 2 to 5 carbon atoms, a phenyl radical, a cyano radical or a

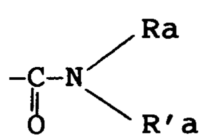

radical in which Ra and R'a, identical or different, represent a hydrogen atom or an alkyl radical containing 1 to 5 carbon atoms, a product (V) as defined above is reacted with a product of formula (XII):

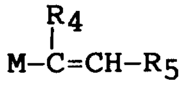

(XII)

in which M, $R_4$ and $R_5$ have the meaning already indicated, in order to obtain a product of formula (XI):

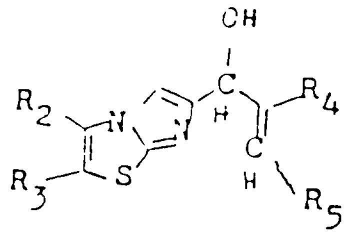

(XI)

in which $R_2$, $R_3$, $R_4$ and $R_5$ have the meaning already indicated, in order to oxidize the product of formula (XI), in order to obtain a product of formula (X):

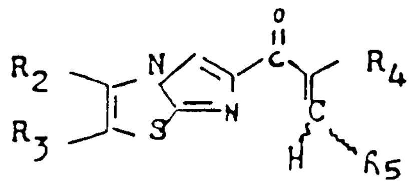

(X)

in which $R_2$, $R_3$, $R_4$ and $R_5$ have the meaning already indicated, then the latter is reacted with an appropriate cyclization agent, in order to obtain a product of formula ($I_B$) in which $R_2$, $R_3$, $R_4$ and $R_5$ have the meaning already indicated and $R'_6$ represents a hydrogen atom, a halogen atom, an alkyl radical containing 1 to 5 carbon atoms, an alkoxycarbonyl radical containing 2 to 5 carbon atoms or a phenyl radical, then if appropriate the product obtained in which $R'_6$ represents an alkoxycarbonyl radical is saponified in order to obtain a product of formula (XIV):

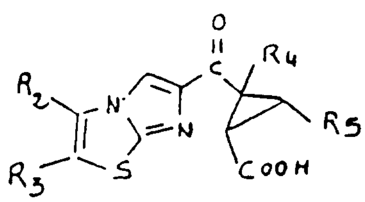

(XIV)

in which $R_2$, $R_3$, $R_4$ and $R_5$ have the meaning already indicated, then the said product of formula (XIV) is reacted with an amine of formula (XIII):

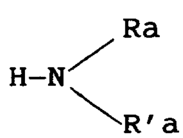

(XIII)

in which Ra and R'a have the meaning already indicated in order to obtain the corresponding product of formula ($I_B$) in which $R'_6$ represents a

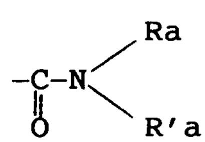

group, then if appropriate, the latter in which Ra and R'a represent a hydrogen atom is dehydrated in

order to obtain the corresponding product of formula ($I_B$) in which $R'_6$ represents a cyano radical, and if appropriate the products of formula ($I_B$) thus obtained are salified.

**2.** Process according to claim 1 for the preparation of products corresponding to formula (I)in which $R_1$ represents either a group (A) in which $R_4$, $R_5$ and $R_6$ represent a hydrogen atom, or a group of formula (B) in which $R_7$ and $R_8$ represent a hydrogen atom and $R_2$ and $R_3$ together represent a group:

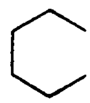

or

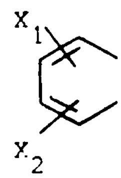

in which $X_1$ and $X_2$, identical or different, represent a hydrogen atom, a methyl radical, a methoxy radical, an isopropoxy radical or a benzyloxy radical, as well as their addition salts with mineral or organic acids, characterized in that there is used at the start a compound of formula (VIII) and (IX) in which $R_2$ and $R_3$ have the meaning indicated above and a compound of formula (VI) in which $R'_1$ represents a group of formula (A) in which $R_4$, $R_5$ and $R_6$ represent a hydrogen atom or a group of formula (B) in which $R_7$ and $R_8$ represent a hydrogen atom or a compound of formula (XII) in which $R_4$ and $R_5$ represent a hydrogen atom and an appropriate cyclization reagent.

**3.** Process according to claim 1 or 2 for the preparation of products corresponding to formula (I) in which $R_1$ represents a group of formula (A) in which $R_4$, $R_5$ and $R_6$ represent a hydrogen atom and $R_2$ and $R_3$ together represent a group:

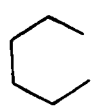

or

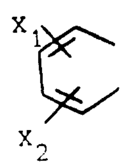

in which $X_1$ represents a hydrogen atom and $X_2$ represents a methoxy radical, an isopropoxy radical or a benzyloxy radical, as well as their addition salts with mineral or organic acids, characterized in that there is used at the start a compound of formula (VIII) or (IX) in which $R_2$ and $R_3$ have the meaning indicated above and a compound of formula (VI) in which $R'_1$ represents a group of formula (A) in which $R_4$, $R_5$ and $R_6$ represent a hydrogen atom or a compound of formula (XII) in which $R_4$ and $R_5$ represent a hydrogen atom and an appropriate cyclization agent.

**Claims for the following Contracting State : GR**

**1.** Preparation process for new imidazo [2,1-b] benzothiazoles corresponding to general formula (I):

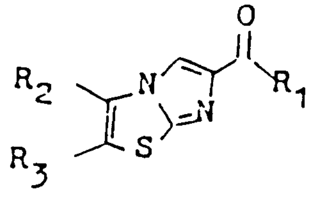

(I)

in which $R_1$ represents a group of formula (A):

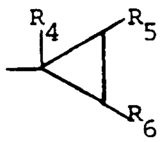

(A)

in which $R_4$ represents a hydrogen atom or an alkyl radical containing 1 to 5 carbon atoms, $R_5$ represents a hydrogen atom or an alkyl radical containing 1 to 5 carbon atoms, $R_6$ represents a hydrogen atom, an alkyl radical containing 1 to 5 carbon atoms, a phenyl radical, a halogen atom, an alkoxycarbonyl radical containing 2 to 5 carbon atoms, a cyano radical, an amido radical, a mono- or dialkylamido radical the alkyl groups of which contain 1 to 5 carbon atoms or $R_1$ represents a group of formula (B):

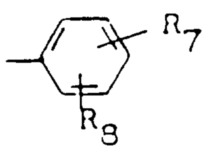

(B)

in which $R_7$ and $R_8$, identical or different, represent a hydrogen atom, a linear or branched alkyl radical containing 1 to 5 carbon atoms, $R_2$ and $R_3$ represent a group:

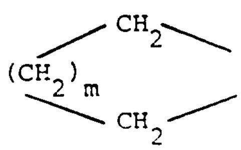

or

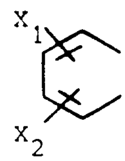

in which m represents the number 1, 2 or 3, $X_1$ and $X_2$, identical or different, represent a hydrogen atom, a linear or branched alkyl radical containing 2 to 5 carbon atoms, a linear or branched alkoxy radical containing 1 to 5 carbon atoms, an aralkoxy radical, an aryloxy radical, a halogen atom, a nitrile or azido group, or $X_1$ and $X_2$ together form a methylenedioxy radical, as well as their addition salts with mineral or organic acids, characterized in that:

a) in order to prepare products corresponding to formula ($I_A$):

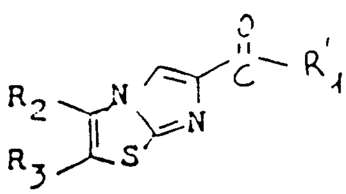

($I_A$)

in which $R_2$ and $R_3$ have the meaning already indicated and $R'_1$ represents a group of formula (A) in which $R_4$ represents a hydrogen atom and $R_5$ or $R_6$ represent a hydrogen atom or an alkoxyl radical containing 1 to 5 carbon atoms or $R'_1$ represents a group of formula (B) in which $R_7$ and $R_8$ have the meaning indicated, either a product of formula (VIII):

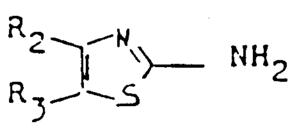

(VIII)

in which $R_2$ and $R_3$ have the meaning already indicated, is reacted with 3-bromo 1-hydroxypran-2-one in order to obtain a product of formula (VII):

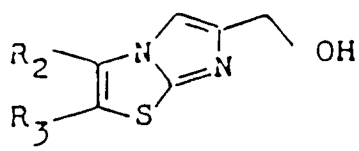

(VII)

in which $R_2$ and $R_3$ have the meaning already indicated, or a product of formula (IX):

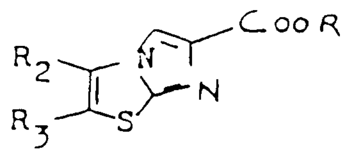

(IX)

in which $R_2$ and $R_3$ have the meaning already indicated and R represents an alkyl radical containing 1 to 3 carbon atoms, is reduced in order to obtain a product of formula (VII) as defined above, which product of formula (VII) is subjected to an oxidizing agent in order to obtain a product of formula (V):

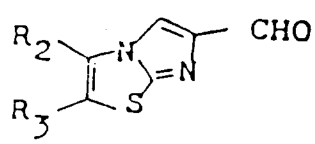

(V)

in which $R_2$ and $R_3$ have the meaning already indicated, which is reacted with a product of formula (VI):

$M\text{-}R'_1$ (VI)

in which M represents a lithium alkali metal atom or a - Mg Hal remainder in which Hal represents a chlorine, bromine or iodine atom and $R_1$ has the meaning already indicated, in order to obtain a product of formula (IV):

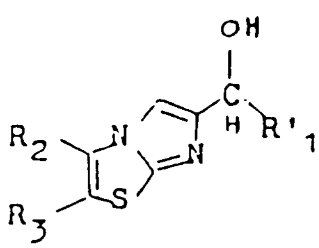

(IV)

in which $R'_1$, $R_2$ and $R_3$ have the meaning already indicated, then the product of formula (IV) thus obtained is oxidized in order to obtain the product of formula ($I_A$) which can be salified if appropriate;

b) in order to prepare products corresponding to formula ($I_B$):

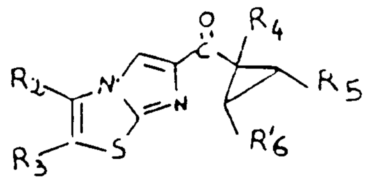

($I_B$)

in which $R_2$, $R_3$, $R_4$ and $R_5$ have the meaning already indicated and $R'_6$ represents a hydrogen atom, a halogen atom, an alkyl radical containing 1 to 5 carbon atoms, an alkoxycarbonyl radical containing 2 to 5 carbon atoms, a phenyl radical, a cyano radical or a

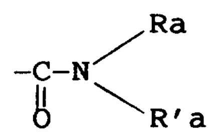

radical in which Ra and R'a, identical or different, represent a hydrogen atom or an alkyl radical containing 1 to 5 carbon atoms, a product (V) as defined above is reacted with a product of formula (XII):

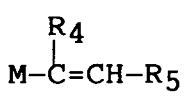

(XII)

in which M, $R_4$ and $R_5$ have the meaning already indicated, in order to obtain a product of formula (XI):

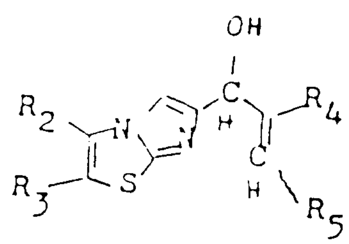

(XI)

in which $R_2$, $R_3$, $R_4$ and $R_5$ have the meaning already indicated, in order to oxidize the product of formula (XI), in order to obtain a product of formula (X):

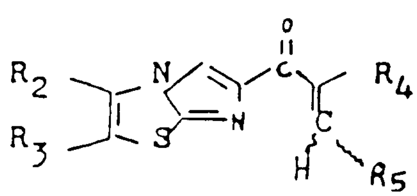

(X)

in which $R_2$, $R_3$, $R_4$ and $R_5$ have the meaning already indicated, then the latter is reacted with an appropriate cyclization agent, in order to obtain a product of formula ($I_B$) in which $R_2$, $R_3$, $R_4$ and $R_5$ have the meaning already indicated and $R'_6$ represents a hydrogen atom, a halogen atom, an alkyl radical containing 1 to 5 carbon atoms, an alkoxycarbonyl radical containing 2 to 5 carbon atoms or

a phenyl radical, then if appropriate the product obtained in which $R'_6$ represents an alkoxycarbonyl radical is saponified in order to obtain a product of formula (XIV):

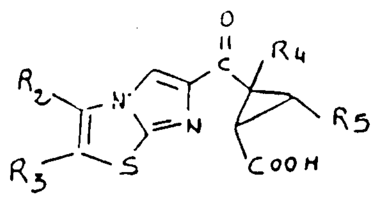

(XIV)

in which $R_2$, $R_3$, $R_4$ and $R_5$ have the meaning already indicated, then the said product of formula (XIV) is reacted with an amine of formula (XIII):

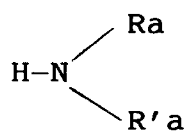

(XIII)

in which Ra and R'a have the meaning already indicated in order to obtain the corresponding product of formula ($I_B$) in which $R'_6$ represents a

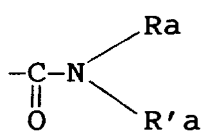

group, then if appropriate, the latter in which Ra and R'a represent a hydrogen atom is dehydrated in order to obtain the corresponding product of formula ($I_B$) in which $R'_6$ represents a cyano radical, and if appropriate the products of formula ($I_B$) thus obtained are salified.

**2.** Process according to claim 1 for the preparation of products corresponding to formula (I)in which $R_1$ represents either a group (A) in which $R_4$, $R_5$ and $R_6$ represent a hydrogen atom, or a group of formula (B) in which $R_7$ and $R_8$ represent a hydrogen atom and $R_2$ and $R_3$ together represent a group:

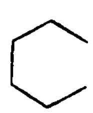

or

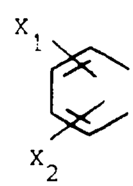

in which $X_1$ and $X_2$, identical or different, represent a hydrogen atom, a methyl radical, a methoxy radical, an isopropoxy radical or a benzyloxy radical, as well as their addition salts with mineral or organic acids, characterized in that there is used at the start a compound of formula (VIII) and (IX) in which $R_2$ and $R_3$ have the meaning indicated above and a compound of formula (VI) in which $R'_1$ represents a group of formula (A) in which $R_4$, $R_5$ and $R_6$ represent a hydrogen atom or a group of formula (B) in which $R_7$ and $R_8$ represent a hydrogen atom or a compound of formula (XII) in which $R_4$ and $R_5$ represent a hydrogen atom and an appropriate cyclization reagent.

**3.** Process according to claim 1 or 2 for the preparation of products corresponding to formula (I) in which $R_1$ represents a group of formula (A) in which $R_4$, $R_5$ and $R_6$ represent a hydrogen atom and $R_2$ and $R_3$ together represent a group:

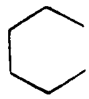

or

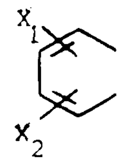

in which $X_1$ represents a hydrogen atom and $X_2$ represents a methoxy radical, an isopropoxy radical or a benzyloxy radical, as well as their addition salts with mineral or organic acids, characterized in that there is used at the start a compound of formula (VIII) or (IX) in which $R_2$ and $R_3$ have the meaning indicated above and a compound of formula (VI) in which $R'_1$ represents a group of formula (A) in which $R_4$, $R_5$ and $R_6$ represent a hydrogen atom or a compound of formula (XII) in which $R_4$ and $R_5$ represent a hydrogen atom and an appropriate cyclization agent.

**4.** As new industrial products, the products of formula (IV), (XI), (X) and (XIV) as defined in claim 1.

**5.** Preparation process for pharmaceutical compositions, characterized in that at least one of the compounds of formula (I) as defined in claim 1 or at least one of their addition salts with pharmaceutically acceptable acids are used as active ingredient in a form intended for this use.

**Patentansprüche**
**Patentansprüche für folgende Vertragsstaaten : AT, BE, CH, DE, FR, IT, LI, LU, NL, SE**

**1.** Neue Imidazo [2,1-b]benzothiazole der allgemeinen Formel (I)

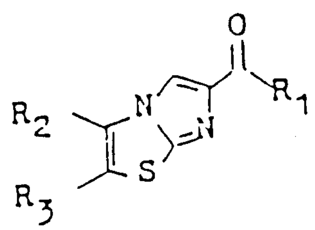

(I)

worin $R_1$ für eine Gruppe der Formel (A)

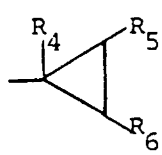

(A)

steht, worin $R_4$ ein Wasserstoffatom oder eine Alkylgruppe mit 1 bis 5 Kohlenstoffatomen bedeutet, $R_5$ ein Wasserstoffatom oder einen Alkylrest mit 1 bis 5 Kohlenstoffatomen wiedergibt, $R_6$ ein Wasserstoffatom, eine Alkylgruppe mit 1 bis 5 Kohlenstoffatomen, eine Phenylgruppe, ein Halogenatom, einen Alkoxycarbonylrest mit 2 bis 5 Kohlenstoffatomen, eine Cyanogruppe, eine Amidogruppe, einen Mono- oder Dialkylamidorest, dessen Alkylgruppen 1 bis 5 Kohlenstoffatome aufweisen, darstellt, oder $R_1$ für eine Gruppe der Formel (B)

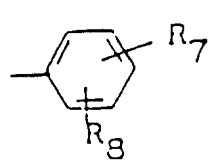

(B)

steht, worin $R_7$ und $R_8$, identisch oder voneinander verschieden, ein Wasserstoffatom, einen linearen oder verzweigten Alkylrest mit 1 bis 5 Kohlenstoffatomen bedeuten, $R_2$ und $R_3$ für eine Gruppe

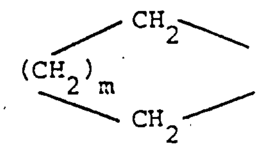

oder

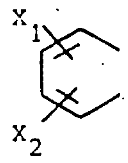

stehen, worin m die Zahl 1, 2 oder 3 bedeutet, $X_1$ und $X_2$, identisch oder voneinander verschieden, für ein Wasserstoffatom, einen linearen oder verzweigten Alkylrest mit 2 bis 5 Kohlenstoffatomen, eine lineare oder verzweigte Alkoxygruppe mit 1 bis 5 Kohlenstoffatomen, einen Aralkoxyrest, einen Aryloxyrest, ein Halogenatom, eine Nitril- oder Azidogruppe stehen oder $X_1$ und $X_2$ gemeinsam einen Methylendioxyrest bilden, sowie deren Additionssalze mit Mineral- oder organischen Säuren.

**2.** Neue Imidazo[2,1-b]benzothiazole der Formel (I) gemäß Anspruch 1, dadurch gekennzeichnet, daß in der Formel (I) $R_1$ entweder eine Gruppe der Formel (A), worin $R_4$, $R_5$ und $R_6$ ein Wasserstoffatom bedeuten, oder eine Gruppe der Formel (B), worin $R_7$ und $R_8$ ein Wasserstoffatom darstellen und $R_2$ und $R_3$ gemeinsam eine Gruppe

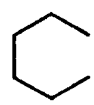

oder

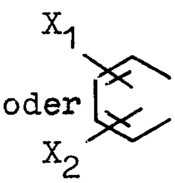

bilden, worin $X_1$ und $X_2$, identisch oder voneinander verschieden, ein Wasserstoffatom, eine Methylgruppe, eine Methoxygruppe, einen Isopropoxyrest oder einen Benzyloxyrest bedeuten, darstellt, sowie deren Additionssalze mit Mineral- oder organischen Säuren.

**3.** Neue Imidazo[2,1-b]benzothiazole gemäß Anspruch 1 oder 2, dadurch gekennzeichnet, daß in der Formel (I) $R_1$ für eine Gruppe der Formel (A) steht, worin $R_4$, $R_5$ und $R_6$ ein Wasserstoffatom bedeuten und $R_2$ und $R_3$ gemeinsam eine Gruppe

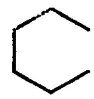

oder

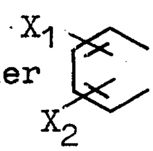

darstellen, in der $X_1$ für ein Wasserstoffatom steht und $X_2$ einen Methoxyrest, einen Isopropoxyrest oder eine Benzyloxygruppe bedeutet, sowie deren Additionssalze mit Mineral- oder organischen Säuren.

**4.** (7-Isopropoxyimidazo[2,1-b]benzothiazol-2-yl)-cyclopropylmethanon und dessen Salze.

**5.** (7-Methoxyimidazo[2,1-b]benzothiazol-2-yl)-cyclopropylmethanon und dessen Salze.

**6.** (7-Benzyloxyimidazo[2,1-b]benzothiazol-2-yl)-cyclopropylmethanon.

**7.** (5,6,7,8-Tetrahydroimidazo[2,1-b]benzothiazol-2-yl)-cyclopropylmethanonund dessen Salze.

**8.** Verfahren zur Herstellung der neuen Imidazo[2,1-b]-benzothiazole der Formel (I) gemäß Anspruch 1, entsprechend der Formel ($I_A$)

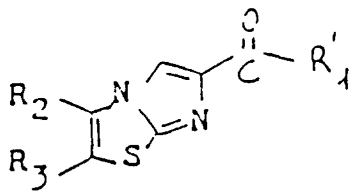

($I_A$)

worin $R_2$ und $R_3$ die vorstehend angegebene Bedeutung besitzen und $R'_1$ eine Gruppe der Formel (A) wiedergibt, worin $R_4$ für ein Wasserstoffatom steht und $R_5$ oder $R_6$ ein Wasserstoffatom oder eine Alkoxygruppe mit 1 bis 5 Kohlenstoffatomen bedeuten, oder $R'_1$ eine Gruppe der Formel (B) wiedergibt, worin $R_7$ und $R_8$ die angegebene Bedeutung besitzen,dadurch gekennzeichnet, daß man entweder ein Produkt der Formel (VIII)

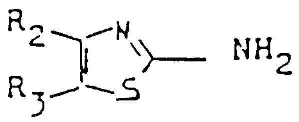

(VIII)

worin $R_2$ und $R_3$ die angegebene Bedeutung besitzen, mit 3-Brom-1-hydroxypyran-2-on umsetzt, um zu einem Produkt der Formel (VII)

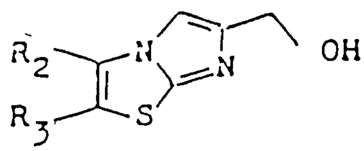

(VII)

zu gelangen, worin $R_2$ und $R_3$ die angegebene Bedeutung haben, oder man ein Produkt der Formel (IX)

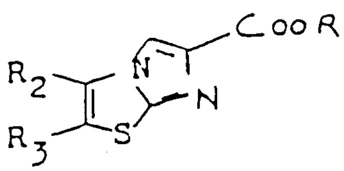

(IX)

worin $R_2$ und $R_3$ die angegebene Bedeutung besitzen und R für eine Alkylgruppe mit 1 bis 3 Kohlenstoffatomen steht, reduziert, um zu einem Produkt der Formel (VII), wie vorstehend definiert, zu gelangen, welches Produkt der Formel (VII) man einer Oxidation unterzieht, um zu einem Produkt der Formel (V)

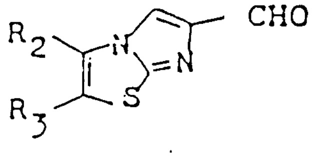

(V)

worin $R_2$ und $R_3$ die angegebene Bedeutung besitzen, zu gelangen, welches man mit einem Produkt der Formel (VI)

$M\text{-}R'_1$ (VI)

worin M ein Atom des Alkalimetalls Lithium oder einen Rest -Mg Hal bedeutet, worin Hal für ein Chlor-, Brom- oder Jodatom steht, und $R_1$ die angegebene Bedeutung besitzt, umsetzt, um zu einem Produkt der Formel (IV)

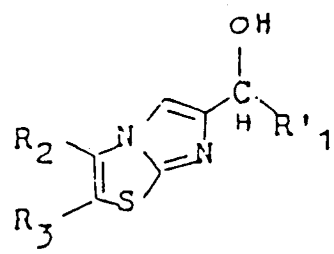

(IV)

worin $R'_1$, $R_2$ und $R_3$ die angegebene Bedeutung besitzen, zu gelangen, wonach man das so erhaltene Produkt der Formel (IV) oxidiert, um zu dem Produkt der Formel ($I_A$) zu gelangen, welches man gegebenenfalls in ein Salz überführt.

**9.** Verfahren gemäß Anspruch 8, dadurch gekennzeichnet, daß

die Umsetzung des Produkts der Formel (V) mit dem Produkt der Formel (VI) unter wasserfreien Bedingungen in einem organischen Lösungsmittel, wie Tetrahydrofuran, durchgeführt wird,

die Oxidation des Produkts der Formel (IV) mit Mangandioxid, Salpetersäure, Ferrichlorid oder Chromoxid in Gegenwart von Pyridin oder auch durch die Oppenauer-Methode oderschließlich durch Dehydrierung in Gegenwart eines Katalysators auf Kupferbasis durchgeführt wird,

die Oxidation des Produkts der Formel (VII) vorteilhaft mit Hilfe von Mangandioxid durchgeführt werden kann,

die Reduktion des Produkts der Formel (IX) vorteilhaft mit Hilfe von Lithiumborhydrid durchgeführt werden kann.

**10.** Verfahren zur Herstellung neuer Imidazo[2,1-b]benzothiazole der Formel (I) gemäß Anspruch 1, entsprechend der Formel ($I_B$)

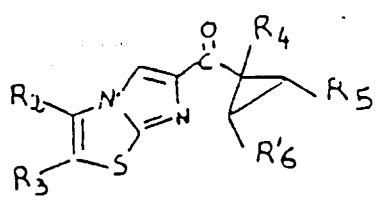

($I_B$)

worin $R_2$, $R_3$, $R_4$ und $R_5$ die angegebene Bedeutung besitzen und $R'_6$ für ein Wasserstoffatom, ein Halogenatom, einen Allylrest mit 1 bis 5 Kohlenstoffatomen, einen Alkoxycarbonylrest mit 2 bis 5 Kohlenstoffatomen, eine Phenylgruppe, eine Cyanogruppe oder eine Gruppe

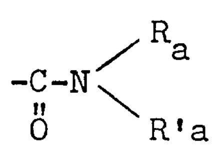

steht, worin Ra und R'a,identisch oder voneinander verschieden, für ein Wasserstoffatom oder einen Alkylrest mit 1 bis 5 Kohlenstoffatomen stehen, dadurch gekennzeichnet, daß man ein Produkt (V), wie vorstehend definiert, mit einem Produkt der Formel (XII)

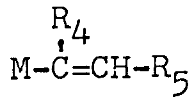

(XII)

worin M, $R_4$ und $R_5$ die angegebene Bedeutung besitzen, umsetzt, um zu einem Produkt der Formel (XI)

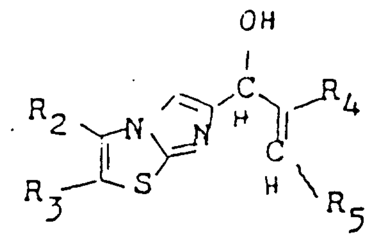

(XI)

worin $R_2$, $R_3$, $R_4$ und $R_5$ die angegebene Bedeutung besitzen, zu gelangen, um das Produkt der Formel (XI) zu oxidieren, um zu einem Produkt der Formel (X)

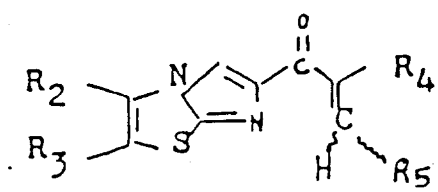

(X)

worin $R_2$, $R_3$, $R_4$ und $R_5$ die angegebene Bedeutung besitzen, zu gelangen, hiernach dieses letztere mit einem geeigneten Cyclisierungsmittel umsetzt, um ein Produkt der Formel ($I_B$), worin $R_2$, $R_3$, $R_4$ und, $R_5$ die angegebene Bedeutung besitzen und $R'_6$ für ein Wasserstoffatom, ein Halogenatom, einen Allrylrest mit 1 bis 5 Kohlenstoffatomen, einen Alkoxycarbonylrest mit 2 bis 5 Kohlenstoffatomen oder einen Phenylrest steht, zu erhalten, wonach man gegebenenfalls das erhaltene Produkt, in dem $R'_6$ für eine Alkoxycarbonylgruppe steht, verseift, um ein Produkt der Formel (XIV)

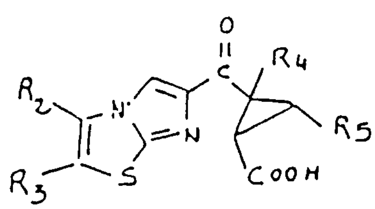

(XIV)

worin $R_2$, $R_3$, $R_4$ und $R_5$ die angegebene Bedeutung besitzen, zu erhalten, wonach man das Produkt der Formel (XIV) mit einem Amin der Formel (XIII)

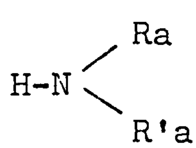

(XIII)

worin Ra und R'a die angegebene Bedeutung besitzen, umsetzt, um zu dem entsprechenden Produkt der Formel ($I_B$), worin $R'_6$ eine Gruppe

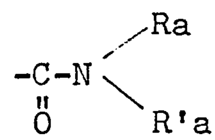

bedeutet, zu gelangen, wonach man gegebenenfalls dieses letztere, in dem Ra und R'a ein Wasserstoffatom bedeuten, dehydratisiert, um das entsprechende Produkt der Formel ($I_B$), worin $R'_6$ eine Cyanogruppe bedeutet, zu erhalten, und gegebenenfalls die so erhaltenen Produkte der Formel ($I_B$) in ein Salz überführt.

**11.** Verfahren gemäß Anspruch 10, dadurch gekennzeichnet, daß

die Umsetzung des Produkts der Formel (V) mit dem Produkt der Formel (XII) unter wasserfreien Bedingungen in einem organischen Lösungsmittel, wie Tetrahydrofuran, durchgeführt wird,

die Oxidation des Produkts der Formel (XI) vorzugsweise mit Mangandioxid, Salpetersäure, Ferrichlorid oder Chromoxid in Gegenwart von Pyridin oder auch durch die Oppenauer-Methode oder schließlich durch Dehydrierung in Gegenwart eines Katalysators auf Kupferbasis durchgeführt wird,

das geeignete Cyclisierungsmittel ein Reagens ist, das in der Lage ist, die Gruppe CH-$R'_6$ im Bereich der Doppelbindung einzuführen,

wenn man ein Produkt der Formel ($I_B$) erhalten möchte, in dem $R_4$ einen Alkylrest mit 1 bis 5 Kohlenstoffatomen bedeutet, $R_5$ ein Wasserstoffatom darstellt und $R'_6$ ein Wasserstoffatom oder eine Alkylgruppe mit 1 bis 5 Kohlenstoffatomen bedeutet, die Cyclisierung vorteilhaft mit Hilfe von Trialkyloxosulfoniumjodid durchgeführt wird, wobei man in dem Medium eines organischen Lösungsmittels, wie Dimethylformamid, arbeitet,

wenn man ein Produkt der Formel ($I_B$) erhalten möchte, worin $R_4$ ein Wasserstoffatom bedeutet, $R_5$ für einen Alkylrest mit 1 bis 3 Kohlenstoffatomen steht und $R'_6$ ein Wasserstoffatom oder eine Alkylgruppe mit 1 bis 5 Kohlenstoffatomen wiedergibt, die Cyclisierung vorteilhaft mit Hilfe von Dimethylaminoalkylphenyloxosulfonium-tetrafluoroborat durchgeführt wird, wobei man in dem Medium eines organischen Lösungsmittels, wie Dimethylformamid, arbeitet,

wenn man ein Produkt der Formel ($I_B$) erhalten möchte, worin $R_4$ und $R_5$ ein Wasserstoffatom bedeuten und $R'_6$ für eine Alkoxycarbonylgruppe steht, die Cyclisierung vorteilhaft mit Hilfe eines Alkyldimethylsulfuranylidenacetats oder eines Acetats eines Benzyldimethylsulfoniumanions durchgeführt wird, wobei man in dem Medium eines organischen Lösungsmittels, wie Chloroform, arbeitet,

wenn man ein Produkt der Formel ($I_B$) erhalten möchte, worin $R_4$ und $R_5$ ein Wasserstoffatom bedeuten und $R'_6$ für ein Halogenatom steht, die Cyclisierung vorteilhaft mit Hilfe eines Halogenodimethylaminophenylsulfoniummethylylidens durchgeführt wird, wobei man in dem Medium eines organischen Lösungsmittels, wie Dimethylformamid, arbeitet,

die Verseifung des Produkts der Formel ($I_B$), worin $R'_6$ einen Alkoxycarbonylrest bedeutet, vorzugsweise mit Hilfe eines Alkalihydroxids, wie Natriumhydroxid, durchgeführt wird,

die Umsetzung des Produkts der Formel (XIV) mit dem Amin der Formel (XII) vorzugsweise in dem Medium eines wasserfreien, organischen Lösungsmittels in Anwesenheit von Carbonyldiimidazol durchgeführt wird,

die Dehydratation des Produkts der Formel ($I_B$) vorzugsweise mit Hilfe eines Anhydrids einer starken Säure, wie Trifluoressigsäureanhydrid, in dem Medium eines organischen Lösungsmittels, wie Methylenchlorid, durchgeführt wird.

**12.** Arzneimittel, dadurch gekennzeichnet, daß sie aus den neuen Imidazo[2,1-b]benzothiazolen der Formel (I) gemäß Anspruch 1 sowie aus deren pharmazeutisch verträglichen Salzen bestehen.

**13.** Arzneimittel, dadurch gekennzeichnet, daß sie aus den neuen Imidazo[2,1-b]benzothiazolen gemäß einem der Ansprüche 2 bis 8 sowie aus deren pharmazeutisch verträglichen Salzen bestehen.

**14.** Pharmazeutische Zusammensetzungen, dadurch gekennzeichnet, daß sie als Wirkstoff zumindest eines der Arzneimittel gemäß Anspruch 12 oder 13 enthalten.

**15.** Als Zwischenprodukte für die Herstellung von Produkten der Formel (I) gemäß Anspruch 1

die Produkte der Formel (IV)

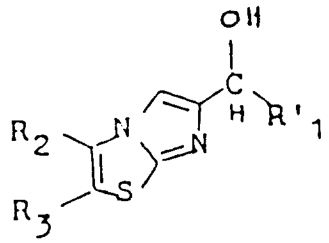

(IV)

worin $R'_1$, $R_2$ und $R_3$ die angegebene Bedeutung besitzen;
die Produkte der Formel (XI)

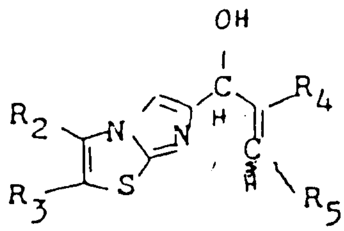

(XI)

worin $R_1$, $R_2$, $R_3$, $R_4$ und $R_5$ die angegebene Bedeutung haben;
die Produkte der Formel (X)

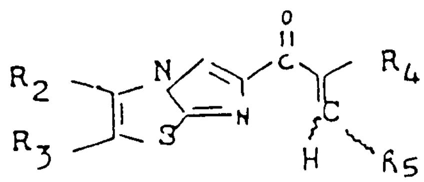

(X)

worin $R_2$, $R_3$, $R_4$ und $R_5$ die angegebene Bedeutung besitzen;
die Produkte der Formel (XIV)

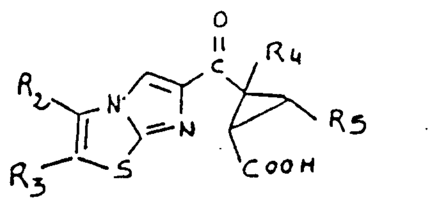

(XIV)

worin $R_2$, $R_3$, $R_4$ und $R_5$ die angegebene Bedeutung besitzen.

**Patentansprüche für folgenden Vertragsstaat : ES**

1. Verfahren zur Herstellung neuer Imidazo[2,1-b]benzothiazole der allgemeinen Formel (I)

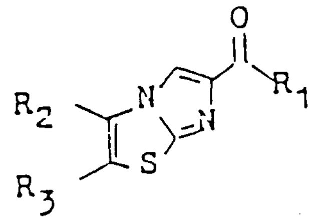

(I)

worin $R_1$ für eine Gruppe der Formel (A)

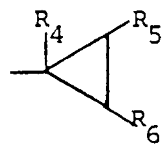

(A)

steht, worin $R_4$ ein Wasserstoffatom oder eine Alkylgruppe mit 1 bis 5 Kohlenstoffatomen bedeutet, $R_5$ ein Wasserstoffatom oder einen Alkylrest mit 1 bis 5 Kohlenstoffatomen wiedergibt, $R_6$ ein Wasserstoffatom, eine Alkylgruppe mit 1 bis 5 Kohlenstoffatomen, eine Phenylgruppe, ein Halogenatom, einen Alkoxycarbonylrest mit 2 bis 5 Kohlenstoffatomen, eine Cyanogruppe, eine Amidogruppe, einen Mono- oder Dialkylamidorest, dessen Alkylgruppen 1 bis 5 Kohlenstoffatome aufweisen, darstellt, oder $R_1$ für eine Gruppe der Formel (B)

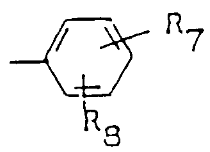

(B)

steht, worin $R_7$ und $R_8$, identisch oder voneinander verschieden, ein Wasserstoffatom, einen linearen oder verzweigten Alkylrest mit 1 bis 5 Kohlenstoffatomen bedeuten, $R_2$ und $R_3$ für eine Gruppe

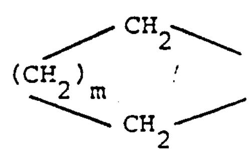

oder

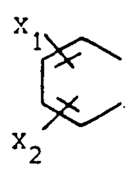

stehen, worin m die Zahl 1, 2 oder 3 bedeutet, $X_1$ und $X_2$, identisch oder voneinander verschieden, für ein Wasserstoffatom, einen linearen oder verzweigten Alkylrest mit 2 bis 5 Kohlenstoffatomen, eine lineare oder verzweigte Alkoxygruppe mit 1 bis 5 Kohlenstoffatomen, einen Aralkoxyrest, einen Aryloxyrest, ein Halogenatom, eine Nitril- oder Azidogruppe stehen oder $X_1$ und $X_2$ gemeinsam einen Methylendioxyrest bilden, sowie von deren Additionssalzen mit Mineral- oder organischen Säuren, dadurch gekennzeichnet, daß man

(a) zur Herstellung der der Formel ($I_A$) entsprechenden Produkte

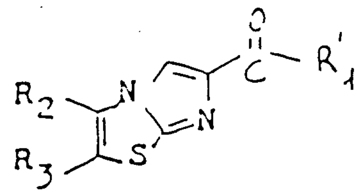

($I_A$)

worin $R_2$ und $R_3$ die vorstehend angegebene Bedeutung besitzen und $R'_1$ eine Gruppe der Formel (A) wiedergibt, worin $R_4$ für ein Wasserstoffatom steht und $R_5$ oder $R_6$ ein Wasserstoffatom oder eine Alkoxygruppe mit 1 bis 5 Kohlenstoffatomen bedeuten, oder $R'_1$ eine Gruppe der Formel (B) wiedergibt, worin $R_7$ und $R_8$ die angegebene Bedeutung besitzen, entweder ein Produkt der Formel (VIII)

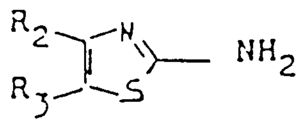

(VIII)

worin $R_2$ und $R_3$ die angegebene Bedeutung besitzen, mit 3-Brom-1-hydroxypyran-2-on umsetzt, um zu einem Produkt der Formel (VII)

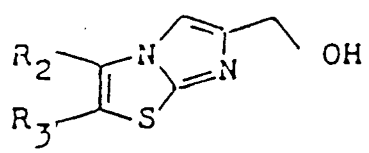

(VII)

zu gelangen, worin $R_2$ und $R_3$ die angegebene Bedeutung haben, oder man ein Produkt der Formel (IX)

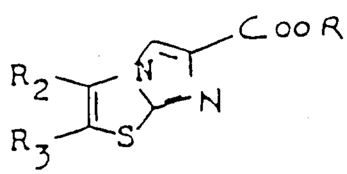

(IX)

worin $R_2$ und $R_3$ die angegebene Bedeutung besitzen und R für eine Alkylgruppe mit 1 bis 3 Kohlenstoffatomen steht, reduziert, um zu einem Produkt der Formel (VII), wie vorstehend definiert, zu gelangen, welches Produkt der Formel (VII) man einer Oxidation unterzieht, um zu einem Produkt der Formel (V)

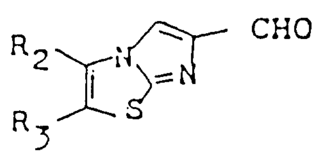

(V)

worin $R_2$ und $R_3$ die angegebene Bedeutung besitzen, zu gelangen, welches man mit einem Produkt der Formel (VI)

M-R'$_1$ (VI)

worin M ein Atom des Alkalimetalls Lithium oder einen Rest -Mg Hal bedeutet, worin Hal für ein Chlor-, Brom- oder Jodatom steht, und $R_1$ die angegebene Bedeutung besitzt, umsetzt, um zu einem Produkt der Formel (IV)

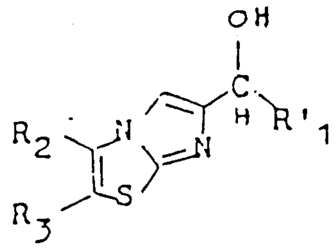

(IV)

worin R'$_1$, $R_2$ und $R_3$ die angegebene Bedeutung besitzen, zu gelangen, wonach man das so erhaltene Produkt der Formel (IV) oxidiert, um zu dem Produkt der Formel ($I_A$) zu gelangen, welches man gegebenenfalls in ein Salz überführt;
(b) zur Herstellung der der Formel ($I_B$) entsprechenden Produkte

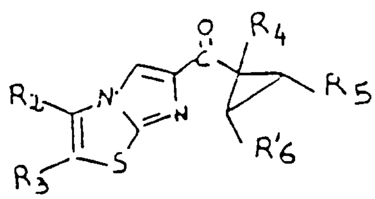

($I_B$)

worin $R_2$, $R_3$, $R_4$ und $R_5$ die angegebene Bedeutung besitzen und R'$_6$ für ein Wasserstoffatom, ein Halogenatom, einen Alkylrest mit 1 bis 5 Kohlenstoffatomen, einen Alkoxycarbonylrest mit 2 bis 5 Kohlenstoffatomen, eine Phenylgruppe, eine Cyanogruppe oder eine Gruppe

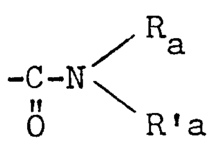

steht, worin Ra und R'a,identisch oder voneinander verschieden, für ein Wasserstoffatom oder einen Alkylrest mit 1 bis 5 Kohlenstoffatomen stehen, ein Produkt (V), wie vorstehend definiert, mit einem Produkt der Formel (XII)

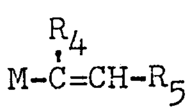

(XII)

worin M, $R_4$ und $R_5$ die angegebene Bedeutung besitzen, umsetzt, um zu einem Produkt der Formel (XI)

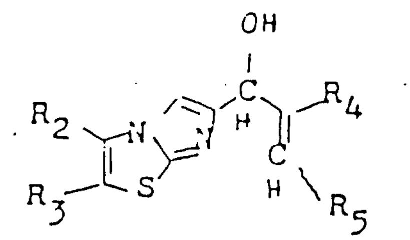

(XI)

worin $R_2$, $R_3$, $R_4$ und $R_5$ die angegebene Bedeutung besitzen, zu gelangen, um das Produkt der Formel (XI) zu oxidieren, um zu einem Produkt der Formel (X)

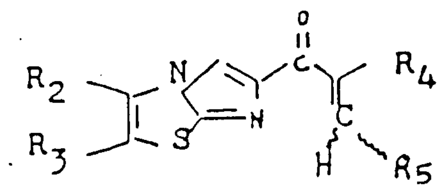

(X)

worin $R_2$, $R_3$, $R_4$ und $R_5$ die angegebene Bedeutung besitzen, zu gelangen, hiernach dieses letztere mit einem geeigneten Cyclisierungsmittel umsetzt, um ein Produkt der Formel ($I_B$), worin $R_2$, $R_3$, $R_4$ und $R_5$ die angegebene Bedeutung besitzen und $R'_6$ für ein Wasserstoffatom, ein Halogenatom, einen Alkylrest mit 1 bis 5 Kohlenstoffatomen, einen Alkoxycarbonylrest mit 2 bis 5 Kohlenstoffatomen oder einen Phenylrest steht, zu erhalten, wonach man gegebenenfalls das erhaltene Produkt, in dem $R'_6$ für eine Alkoxycarbonylgruppe steht, verseift, um ein Produkt der Formel (XIV)

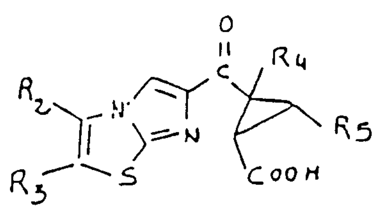

(XIV)

worin $R_2$, $R_3$, $R_4$ und $R_5$ die angegebene Bedeutung besitzen, zu erhalten, wonach man das Produkt der Formel (XIV) mit einem Amin der Formel (XIII)

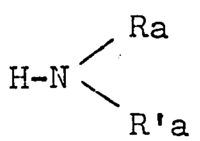

(XIII)

worin Ra und R'a die angegebene Bedeutung besitzen, umsetzt, um zu dem entsprechenden Produkt der Formel ($I_B$), worin $R'_6$ eine Gruppe

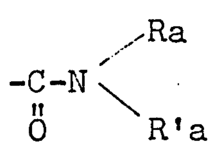

bedeutet, zu gelangen, wonach man gegebenenfalls dieses letztere, in dem Ra und R'a ein Wasserstoffatom bedeuten, dehydratisiert, um das entsprechende Produkt der Formel ($I_B$), worin $R'_6$ eine Cyanogruppe bedeutet, zu erhalten, und gegebenenfalls die so erhaltenen Produkte der Formel ($I_B$) in ein Salz überführt.

**2.** Verfahren gemaß Anspruch 1 zur Herstellung der der Formel (I) entsprechenden Produkte, worin $R_1$ entweder eine Gruppe (A), in der $R_4$, $R_5$ und $R_6$ ein Wasserstoffatom bedeuten,oder eine Gruppe der Formel (B) darstellt, worin $R_7$ und $R_8$ ein Wasserstoffatom bedeuten und $R_2$ und $R_3$ gemeinsam eine Gruppe

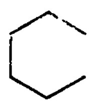 oder 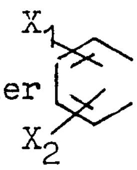

darstellen, worin $X_1$ und $X_2$, identisch oder voneinander verschieden, ein Wasserstoffatom, eine Methylgruppe, eine Methoxygruppe, eine Isopropoxygruppe oder eine Benzyloxygruppe bedeuten, sowie von deren Additionssalzen mit Mineral- oder organischen Säuren, dadurch gekennzeichnet, daß man von einer Verbindung der Formel (VIII) und (IX), worin $R_2$ und $R_3$ die vorstehend angegebene Bedeutung besitzen, und einer Verbindung der Formel (VI), worin $R'_1$ eine Gruppe der Formel (A), in der $R_4$, $R_5$ und $R_6$ ein Wasserstoffatom wiedergeben, oder eine Gruppe der Formel (B) darstellt, worin $R_7$ und $R_8$ ein Wasserstoffatom bedeuten, oder von einer Verbindung der Formel (XII), worin $R_4$ und $R_5$ ein Wasserstoffatom bedeuten, und einem geeigneten Cyclisierungsreagens ausgeht.

**3.** Verfahren gemäß Anspruch 1 oder 2 zur Herstellung der der Formel (I) entsprechenden Produkte, worin $R_1$ eine Gruppe der Formel (A) darstellt, in der $R_4$, $R_5$ und $R_6$ ein Wasserstoffatom bedeuten und $R_2$ und $R_3$ gemeinsam eine Gruppe

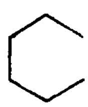 oder 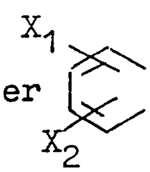

bilden, worin $X_1$ für ein Wasserstoffatom steht und $X_2$ eine Methoxygruppe, eine Isopropoxygruppe oder eine Benzyloxygruppe darstellt, sowie von deren Additionssalzen mit Mineral- oder organischen Säuren,
dadurch gekennzeichnet, daß man von einer Verbindung der Formel (VIII) oder (IX), worin $R_2$ und $R_3$ die vorstehend angegebene Bedeutung besitzen, und einer Verbindung der Formel (VI), worin $R'_1$ eine Gruppe der Formel (A), in der $R_4$, $R_5$ und $R_6$ ein Wasserstoffatom bedeuten, darstellt, oder von einer Verbindung der Formel (XII), worin $R_4$ und $R_5$ ein Wasserstoffatom bedeuten, und einem geeigneten Cyclisierungsreagens ausgeht.

**Patentansprüche für folgenden Vertragsstaat : GR**

**1.** Verfahren zur Herstellung neuer Imidazo [2,1-b]benzothiazole der allgemeinen Formel (I)

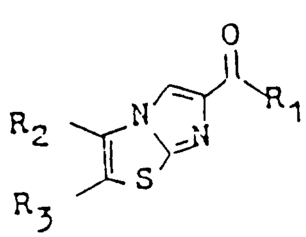

(I)

worin $R_1$ für eine Gruppe der Formel (A)

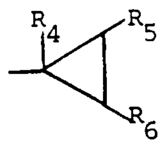

(A)

steht, worin $R_4$ ein Wasserstoffatom oder eine Alkylgruppe mit 1 bis 5 Kohlenstoffatomen bedeutet, $R_5$ ein Wasserstoffatom oder einen Alkylrest mit 1 bis 5 Kohlenstoffatomen wiedergibt, $R_6$ ein Wasserstoffatom, eine Alkylgruppe mit 1 bis 5 Kohlenstoffatomen, eine Phenylgruppe, ein Halogenatom, einen Alkoxycarbonylrest mit 2 bis 5 Kohlenstoffatomen, eine Cyanogruppe, eine Amidogruppe, einen Mono- oder Dialkylamidorest, dessen Alkylgruppen 1 bis 5 Kohlenstoffatome aufweisen, darstellt, oder $R_1$ für eine Gruppe der Formel (B)

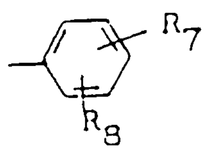

(B)

steht, worin $R_7$ und $R_8$, identisch oder voneinander verschieden, ein Wasserstoffatom, einen linearen oder verzweigten Alkylrest mit 1 bis 5 Kohlenstoffatomen bedeuten, $R_2$ und $R_3$ für eine Gruppe

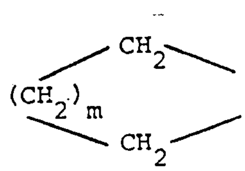

oder

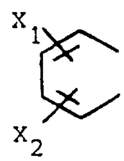

stehen, worin m die Zahl 1, 2 oder 3 bedeutet, $X_1$ und $X_2$, identisch oder voneinander verschieden, für ein Wasserstoffatom, einen linearen oder verzweigten Alkylrest mit 2 bis 5 Kohlenstoffatomen, eine lineare oder verzweigte Alkoxygruppe mit 1 bis 5 Kohlenstoffatomen, einen Aralkoxyrest, einen Aryloxyrest, ein Halogenatom, eine Nitril- oder Azidogruppe stehen oder $X_1$ und $X_2$ gemeinsam einen Methylendioxyrest bilden, sowie von deren Additionssalzen mit Mineral- oder organischen Säuren,
dadurch gekennzeichnet, daß man

(a) zur Herstellung der der Formel ($I_A$) entsprechenden Produkte

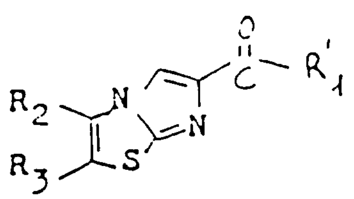

($I_A$)

worin $R_2$ und $R_3$ die vorstehend angegebene Bedeutung besitzen und $R'_1$ eine Gruppe der Formel (A) wiedergibt, worin $R_4$ für ein Wasserstoffatom steht und $R_5$ oder $R_6$ ein Wasserstoffatom oder eine Alkoxygruppe mit 1 bis 5 Kohlenstoffatomen bedeuten, oder $R'_1$ eine Gruppe der Formel (B) wiedergibt, worin $R_7$ und $R_8$ die angegebene Bedeutung besitzen, entweder ein Produkt der Formel (VII)

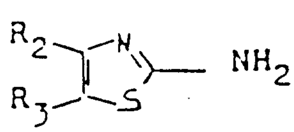

(VIII)

worin $R_2$ und $R_3$ die angegebene Bedeutung besitzen, mit 3-Brom-1-hydroxypyran-2-on umsetzt, um

zu einem Produkt der Formel (VII)

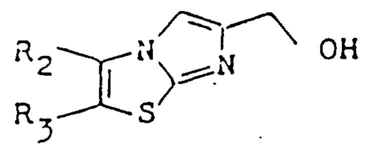

(VII)

zu gelangen, worin $R_2$ und $R_3$ die angegebene Bedeutung haben, oder man ein Produkt der Formel (IX)

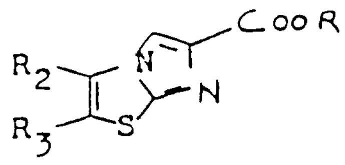

(IX)

worin $R_2$ und $R_3$ die angegebene Bedeutung besitzen und R für eine Alkylgruppe mit 1 bis 3 Kohlenstoffatomen steht, reduziert, um zu einem Produkt der Formel (VII), wie vorstehend definiert, zu gelangen, welches Produkt der Formel (VII) man einer Oxidation unterzieht, um zu einem Produkt der Formel (V)

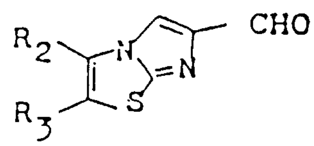

(V)

worin $R_2$ und $R_3$ die angegebene Bedeutung besitzen, zu gelangen, welches man mit einem Produkt der Formel (VI)

$M\text{-}R'_1$ (VI)

worin M ein Atom des Alkalimetalls Lithium oder einen Rest -Mg Hal bedeutet, worin Hal für ein Chlor-, Brom- oder Jodatom steht, und $R_1$ die angegebene Bedeutung besitzt, umsetzt, um zu einem Produkt der Formel (IV)

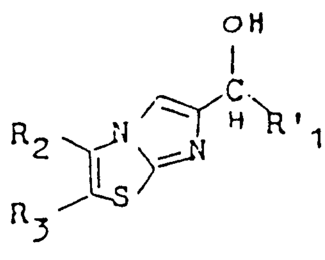

(IV)

worin $R'_1$, $R_2$ und $R_3$ die angegebene Bedeutung besitzen, zu gelangen, wonach man das so erhaltene Produkt der Formel (IV) oxidiert, um zu dem Produkt der Formel ($I_A$) zu gelangen, welches man gegebenenfalls in ein Salz überführt;

(b) zur Herstellung der der Formel ($I_B$) entsprechenden Produkte

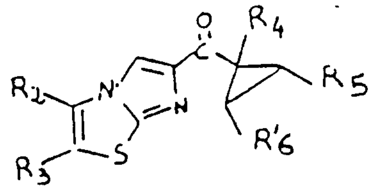

($I_B$)

worin $R_2$, $R_3$, $R_4$ und $R_5$ die angegebene Bedeutung besitzen und $R'_6$ für ein Wasserstoffatom, ein Halogenatom, einen Alkylrest mit 1 bis 5 Kohlenstoffatomen, einen Alkoxycarbonylrest mit 2 bis 5 Kohlenstoffatomen, eine Phenylgruppe, eine Cyanogruppe oder eine Gruppe

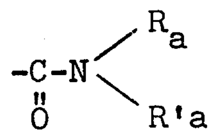

steht, worin Ra und R'a,identisch oder voneinander verschieden, für ein Wasserstoffatom oder einen Alkylrest mit 1 bis 5 Kohlenstoffatomen stehen, ein Produkt (V), wie vorstehend definiert, mit einem Produkt der Formel (XII)

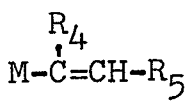

(XII)

worin M, $R_4$ und $R_5$ die angegebene Bedeutung besitzen, umsetzt, um zu einem Produkt der Formel (XI)

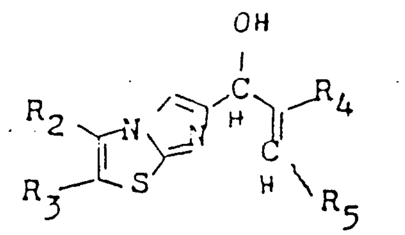

(XI)

worin $R_2$, $R_3$, $R_4$ und $R_5$ die angegebene Bedeutung besitzen, zu gelangen, um das Produkt der Formel (XI) zu oxidieren, um zu einem Produkt der Formel (X)

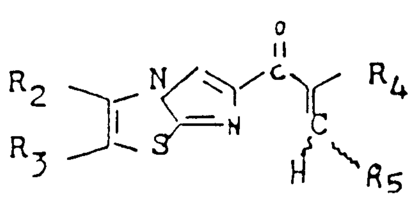

(X)

worin $R_2$, $R_3$, $R_4$ und $R_5$ die angegebene Bedeutung besitzen, zu gelangen, hiernach dieses letztere mit einem geeigneten Cyclisierungsmittel umsetzt, um ein Produkt der Formel ($I_B$), worin $R_2$, $R_3$, $R_4$ und $R_5$ die angegebene Bedeutung besitzen und $R'_6$ für ein Wasserstoffatom, ein Halogenatom, einen Alkylrest mit 1 bis 5 Kohlenstoffatomen, einen Alkoxycarbonylrest mit 2 bis 5 Kohlenstoffatomen oder einen Phenylrest steht, zu erhalten, wonach man gegebenenfalls das erhaltene Produkt, in

dem $R'_6$ für eine Alkoxycarbonylgruppe steht, verseift, um ein Produkt der Formel (XIV)

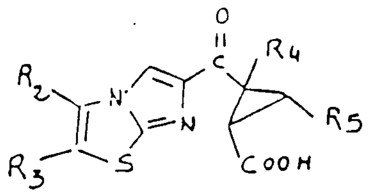

(XIV)

worin $R_2$, $R_3$, $R_4$ und $R_5$ die angegebene Bedeutung besitzen, zu erhalten, wonach man das Produkt der Formel (XIV) mit einem Amin der Formel (XIII)

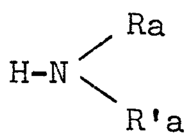

(XIII)

worin Ra und R'a die angegebene Bedeutung besitzen, umsetzt, um zu dem entsprechenden Produkt der Formel ($I_B$), worin $R'_6$ eine Gruppe

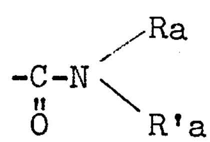

bedeutet, zu gelangen, wonach man gegebenenfalls dieses letztere, in dem Ra und R'a ein Wasserstoffatom bedeuten, dehydratisiert, um das entsprechende Produkt der Formel ($I_B$), worin $R'_6$ eine Cyanogruppe bedeutet, zu erhalten, und gegebenenfalls die so erhaltenen Produkte der Formel ($I_B$) in ein Salz überführt.

**2.** Verfahren gemaß Anspruch 1 zur Herstellung der der Formel (I) entsprechenden Produkte, worin $R_1$ entweder eine Gruppe (A), in der $R_4$, $R_5$ und $R_6$ ein Wasserstoffatom bedeuten,oder eine Gruppe der Formel (B) darstellt, worin $R_7$ und $R_8$ ein Wasserstoffatom bedeuten und $R_2$ und $R_3$ gemeinsam eine Gruppe

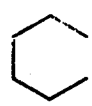 oder 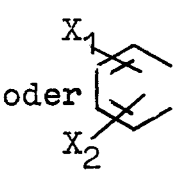

darstellen, worin $X_1$ und $X_2$, identisch oder voneinander verschieden, ein Wasserstoffatom, eine Methylgruppe, eine Methoxygruppe, eine Isopropoxygruppe oder eine Benzyloxygruppe bedeuten, sowie von deren Additionssalzen mit Mineral- oder organischen Säuren, dadurch gekennzeichnet, daß man von einer Verbindung der Formel (VIII) und (IX), worin $R_2$ und $R_3$ die vorstehend angegebene Bedeutung besitzen, und einer Verbindung der Formel (VI), worin $R'_1$ eine Gruppe der Formel (A), in der $R_4$, $R_5$ und $R_6$ ein Wasserstoffatom wiedergeben, oder eine Gruppe der Formel (B) darstellt, worin $R_7$ und $R_8$ ein Wasserstoffatom bedeuten, oder von einer Verbindung der Formel (XII), worin $R_4$ und $R_5$ ein Wasserstoffatom bedeuten, und einem geeigneten Cyclisierungsreagens ausgeht.

**3.** Verfahren gemäß Anspruch 1 oder 2 zur Herstellung der der Formel (I) entsprechenden Produkte, worin $R_1$ eine Gruppe der Formel (A) darstellt, in der $R_4$, $R_5$ und $R_6$ ein Wasserstoffatom bedeuten und $R_2$ und $R_3$ gemeinsam eine Gruppe

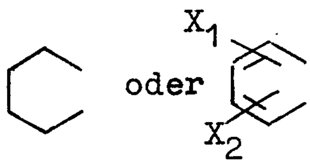

bilden, worin $X_1$ für ein Wasserstoffatom steht und $X_2$ eine Methoxygruppe, eine Isopropoxygruppe oder eine Benzyloxygruppe darstellt, sowie von deren Additionssalzen mit Mineral- oder organischen Säuren,
dadurch gekennzeichnet, daß man von einer Verbindung der Formel (VIII) oder (IX), worin $R_2$ und $R_3$ die vorstehend angegebene Bedeutung besitzen, und einer Verbindung der Formel (VI), worin $R'_1$ eine Gruppe der Formel (A), in der $R_4$, $R_5$ und $R_6$ ein Wasserstoffatom bedeuten, darstellt, oder von einer Verbindung der Formel (XII), worin $R_4$ und $R_5$ ein Wasserstoffatom bedeuten, und einem geeigneten Cyclisierungsreagens ausgeht.

4. Als neue industrielle Produkte die Produkte der Formel (IV), (XI), (X) und (XIV), wie in Anspruch 1 definiert.

5. Verfahren zur Herstellung von pharmazeutischen Zusammensetzungen, dadurch gekennzeichnet, daß man als Wirkstoff zumindest eine der Verbindungen der Formel (I), wie in Anspruch 1 definiert, oder zumindest eines ihrer Additionssalze mit pharmazeutisch verträglichen Säuren in eine für diese Verwendung bestimmte Form überführt.